# EUROPÄISCHE PATENTANMELDUNG

(11) **EP 2 123 619 A1**
(43) Veröffentlichungstag der Anmeldung: **25.11.2009**
(21) Anmeldenummer: 08075508.5
(22) Anmeldetag: 20.05.2008
(51) Int. Cl.: C07B 59/00, C07D 207/28, C07C 229/24, C07C 271/22, A61K 31/225, A61K 51/04, A61P 35/00

(54) **Neue [F-18]-Markierte L-Glutaminsäure-und L-Glutaminderivate (II), ihre Verwendung sowie Verfahren zu ihrer Herstellung**

(71) Anmelder: Bayer Schering Pharma Aktiengesellschaft, 13353 Berlin (DE)
(72) Erfinder: Dr. Lehmann, Lutz, 42329 Wuppertal (DE); Dr. Dinkelborg, Ludger, 13465 Berlin (DE); Dr. Friebe, Mattias, 13509 Berlin (DE); Dr. Anklamer, Keith Graham, 10115 Berlin (DE); Dr. Koglin, Norman, 12587 Berlin (DE); Dr. Berndt, Mathias, 12163 Berlin (DE); Dr. Schmitt-Willich, Heribert, 10551 Berlin (DE)

(57) **Zusammenfassung**

Es werden die Verbindungen und die Synthese von [F-18] markierter L-Glutaminsäure, [F-18] markiertem L-Glutamat, ihren Derivaten gemäß Formel (I) und ihre Verwendungen beschrieben.

## Beschreibung

Die Erfindung betrifft die in den Patentansprüchen gekennzeichneten Gegenstände, nämlich [F-18] markierte L-Glutaminsäurederivate und [F-18] markierte L-Glutaminderivate der allgemeinen Formel I, sowie ihre Verwendung und Verfahren zu ihrer Herstellung.

Die frühe Diagnose von malignen Tumorerkrankungen spielt eine sehr wichtige Rolle für die Überlebensprognose eines Tumorpatienten. Bei dieser Diagnose sind nicht-invasive, bildgebende diagnostische Verfahren ein wichtiges Hilfsmittel. In den letzten Jahren hat sich hier vor allem die PET-Technologie (Positronen-Emissions-Tomographie) als besonders nützlich erwiesen. Die Sensitivität und Spezifität der PET-Technologie hängt wesentlich von der verwendeten signalgebenden Substanz (Tracer) und ihrer Verteilung im Körper ab. Bei der Suche nach geeigneten Tracern versucht man bestimmte Eigenschaften von Tumoren auszunutzen, die Tumorgewebe von gesundem, umliegenden Gewebe unterscheiden. Das bevorzugte kommerziell genutzte Isotop, welches für PET Anwendung findet, ist ¹⁸F. ¹⁸F stellt durch seine kurze Halbwertszeit von unter 2 Stunden besondere Anforderungen an die Herstellung geeigneter Tracer. Aufwändige, lange Synthesewege und Aufreinigungen sind mit diesem Isotop nicht möglich, da sonst ein erheblicher Teil der Radioaktivität des Isotops bereits abgeklungen ist, bevor der Tracer zur Diagnose eingesetzt werden kann. Es ist deshalb häufig nicht möglich etablierte Synthesewege für nichtradioaktive Fluorierungen auf die Synthese von ¹⁸F-Tracern anzuwenden. Des weiteren führt die hohe spezifische Aktivität des ¹⁸F (ca. 80 GBq/nmol) zu sehr niedrigen Substanzmengen an [¹⁸F]Fluorid für die Tracersynthese, was wiederum einen extremen Überschuß an Präkursor bedingt und den Erfolg einer auf nicht-radioaktiven Fluorierungsreaktionen basierender Radiosynthesestrategie unvorhersehbar gestaltet.

FDG ([¹⁸F]2-Fluorodesoxyglukose)-PET ist ein weithin akzeptiertes und verbreitetes Hilfsmittel in der Diagnose und weiteren klinischen Verfolgung von Tumorerkrankungen. Maligne Tumore konkurrieren mit dem Wirtsorganismus um Glukoseversorgung zur Nährstoffversorgung (Warburg O. Über den Stoffwechsel der Carcinomzelle. Biochem. Zeitschrift 1924; 152: 309-339; Kellof G. Progress and Promise of FDG-PET Imaging for Cancer Patient Management and Oncologic Drug Development. Clin Cancer Res. 2005; 11 (8): 2785-2807) Dabei haben Tumorzellen im Vergleich zu umliegenden Zellen des Normalgewebes gewöhnlich einen erhöhten Glukosestoffwechsel. Dies wird bei der Verwendung von Fluorodesoxyglukose (FDG), einem Glukosederivat genutzt, welches verstärkt in die Zellen transportiert wird, dort aber nach der Phosphorylierung als FDG-6-phosphat metabolisch eingeschlossen wird ("Warburg-Effekt"). ¹⁸F markiertes FDG ist daher ein wirkungsvoller Tracer zur Detektion von Tumorerkrankungen beim Patienten mittels der PET-Technologie. Auf der Suche nach neuen PET Tracern wurden in jüngster Zeit auch zunehmend Aminosäuren für die ¹⁸F PET Bildgebung eingesetzt (z. B. (review): Eur J Nucl Med Mol Imaging. 2002 May;29(5):681-90). Dabei eignen sich einige der ¹⁸F markierten Aminosäuren für die Messung der Geschwindigkeitsrate der Proteinsynthese, die meisten anderen Derivate aber für die Messung der direkten Zellaufnahme im Tumor. Bekannte ¹⁸F markierte Aminosäuren sind z. B. von Tyrosin-, Phenylalanin-, Prolin-, Asparagin- und unnatürlichen Aminosäuren abgeleitet (z. B. J. Nucl Med 1991; 32:1338-1346, J Nucl Med 1996; 37:320-325, J Nucl Med 2001;42:752-754 bzw. J Nucl Med 1999; 40:331-338.). Glutaminsäure und Glutamin sind als ¹⁸F markierte Derivate nicht bekannt, wohingegen nicht-radioaktive fluorierte Glutamin- und Glutaminsäurederivate geläufig sind; so z. Bsp. jene, die an γ-Position (z. Bsp. (review): Amino Acids. (2003) Apr;24(3):245-61) oder an β-Position (z. Bsp. Tetrahedron Lett.; 30; 14; 1989; 1799-1802, J. Org. Chem.; 54; 2; 1989; 498-500, Tetrahedron: Asymmetry; 12; 9; 2001; 1303 - 1312) Fluor aufweisen.

Von Glutaminsäurederivaten, die an den chemischen Funktionalitäten Schutzgruppen und in β oder γ Position eine Abgangsgruppe aufweisen, ist in der Vergangenheit bereits berichtet worden. So wurde über Glutamat als Mesylat bzw. Bromid in γ-Position informiert, deren Säure- und Aminfunktionen mit Ester- bzw. Z-Schutzgruppen versehen waren (J. Chem. Soc. Perkin Trans. 1; 1986; 1323-1328) oder beispielsweise von γ-Chloro-Glutaminsäure ohne Schutzgruppen (Synthesis; (1973); 44-46). Über ähnliche Derivate, bei denen aber die Fluchtgruppe in β-Stellung positioniert ist, wurde ebenfalls verschiedentlich berichtet: z. Bsp. Chem. Pharm. Bull.; 17; 5; (1969); 879-885, J.Gen.Chem.USSR (Engl.Transl.); 38; (1968); 1645-1648; Tetrahedron Lett.; 27; 19; (1986); 2143-2144, Chem. Pharm. Bull.; EN; 17; 5; 1969; 873-878, Patent FR 1461184, Patent JP 13142.)

Die derzeitigen PET-Tracer, die für die Tumordiagnostik eingesetzt werden, haben einige unbestrittene Nachteile: So reichert sich FDG zwar bevorzugt in solchen Zellen mit erhöhtem Glukosestoffwechsel an, es gibt allerdings auch in anderen pathologischen und physiologischen Zuständen einen erhöhten Glukosestoffwechsel in den beteiligten Zellen und Geweben, z. Bsp. Infektionsherde oder Wundheilung (zusammengefasst in J. Nucl. Med. Technol. (2005), 33, 145-155). Es ist häufig immer noch schwierig zu entscheiden, ob eine mittels FDG-PET detektierte Läsion tatsächlich neoplastischen Ursprungs ist oder auf andere physiologische oder pathologische Zustände des Gewebes zurückzuführen ist. Insgesamt weist die Diagnosetätigkeit mittels FDG-PET in der Onkologie eine Sensitivität von 84% und eine Spezifität von 88% auf (Gambhir et al. "A tabulated summary of the FDG PET literature" J. Nucl. Med. 2001, 42, 1-93S). Tumore im Gehirn lassen sich beispielsweise durch die hohe Anreicherung von FDG in gesundem Hirngewebe nur sehr schwer darstellen.

Die bisher bekannten ¹⁸F markierten Aminosäurederivate sind in einigen Fällen gut geeignet, um Tumore im Gehirn zu detektieren ((review): Eur J Nucl Med Mol Imaging. 2002 May;29(5):681-90), allerdings können sie bei anderen Tumoren nicht mit den Bildgebungseigenschaften des "Goldstandards" [¹⁸F]2-FDG konkurrieren. Die metabolische Anreicherung und Retention der bislang F-18 markierten Aminosäuren in tumorösem Gewebe ist in der Regel niedriger als für FDG. Darüberhinaus, ist die Zugänglichkeit isomerenreiner F-18 markierter nichtaromatischer Aminosäuren chemisch höchst anspruchsvoll.

Ähnlich wie für Glukose wurde auch für Glutaminsäure und Glutamin ein erhöhter Metabolismus in proliferierenden Tumorzellen beschrieben (Medina, J Nutr. 1131:2539S-2542S, 2001; Souba, Ann Surg 218: 715-728, 1993). Die erhöhte Rate an Protein- und Nukleinsäuresynthesen sowie die Energiegewinnung per se werden als Gründe für einen verstärkten Glutaminkonsum von Tumorzellen angenommen. Die Synthese von entsprechenden C-11 und C-14 markierten, mit dem natürlichen Substrat also identischen Verbindungen, wurde in der Literatur bereits beschrieben (z. Bsp. Antoni, Enzyme Catalyzed Synthesis of L-[4-C-11]Aspartate and L-[5-C-11]Glutamate. J. Labelled Compd. Radiopharm. 44;( 4) 2001: 287 - 294) und Buchanan, The biosynthesis of showdomycin: studies with stable isotopes and the determination of principal precursors. J. Chem. Soc. Chem. Commun.; EN; 22; 1984; 1515-1517). Erste Hinweise mit der C-11 markierten Verbindung deuten auf keine signifikante Tumorakkumulation hin.

Aufgabe der vorliegenden Erfindung ist es neue Verbindungen zu finden, die sich in [¹⁸F] markierter Form für die PET-basierte Diagnose eignen.

### Erfindung

Die Aufgabe wird gelöst durch die erfindungsgemäße Bereitstellung von [¹⁸F] markierten Glutaminsäurederivaten und [¹⁸F] markierten Glutaminderivaten, gemäß der allgemeinen Formel (I), einschließlich ihrer Diastereomere und Enantiomere: 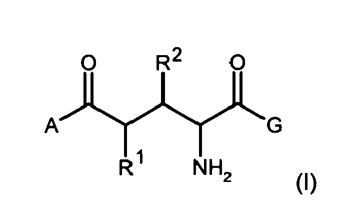 worin
A für
   a) Hydroxyl,
   b) verzweigtes oder unverzweigtes C₁-C₅ Alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ Alkyl)ₙ-O-C₁-C₄ Alkyl,
   e) N(C₁-C₅ Alkyl)₂,
   f) NH₂,
   g) N(H)-L,
   h) O-L oder
   i) O-Z
      steht,
G für
   a) Hydroxyl,
   b) O-Z
   c) verzweigtes oder unverzweigtes O-C₁-C₅ Alkyl,
   d) verzweigtes oder unverzweigtes O-C₂-C₅ Alkenyl,
   e) verzweigtes oder unverzweigtes O-C₁-C₅ Alkyl-(O-C₁-C₄ Alkyl)ₙ-O-C₁-C₄ Alkyl,
   f) verzweigtes oder unverzweigtes 0-C₂-C₅ Alkinyl, oder
   g) Triphenylmethoxy
      steht,
R¹ und R² für
   a) Wasserstoff,
   b) verzweigtes oder unverzweigtes ¹⁸F-C₆-C₁₀ Alkoxy,
   c) verzweigtes oder unverzweigtes ¹⁸F-C₆-C₁₀ Alkyl,
   d) verzweigtes oder unverzweigtes ¹⁸F-C₆-C₁₀ Alkenyl,
   e) verzweigtes oder unverzweigtes ¹⁸F-C₆-C₁₀ Alkinyl,
   f) Hydroxyl,
   g) verzweigtes oder unverzweigtes C₁-C₅ Alkyl oder
   h) verzweigtes oder unverzweigtes C₁-C₅ Alkoxy
      steht,
      mit der Maßgabe, dass einer der Substituenten R¹ oder R² genau ein ¹⁸F Isotop beinhaltet und der jeweils andere Substituent kein ¹⁸F Isotop beinhaltet,
L für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ Alkyl)ₙ-O-C₁-C₄ Alkyl oder
   d) verzweigtes oder unverzweigtes C₂-C₅ Alkinyl,
      steht, und
Z für ein Metallkationenequivalent steht,
wobei
n = 0, 1, 2 oder 3 ist und
wobei alle möglichen Diastereomere und Enantiomere Teil des vorliegenden Erfindungsgegenstandes sind.

Bevorzugte Verbindungen der Erfindung gemäß der Formel (I) zeichnen sich dadurch aus, dass
A für
   a) Hydroxyl,
   b) Methoxy,
   c) Ethoxy,
   d) Propoxy,
   e) NMe₂,
   f) NEt₂ ,
   g) NH₂,
   h) N(H)-L,
   i) O-L oder
   j) O-Z
      steht.

Weitere bevorzugte Verbindungen der Erfindung gemäß der Formel (I) zeichnen sich dadurch aus, dass
A für
   a) Hydroxyl,
   b) Methoxy,
   c) Ethoxy,
   d) NMe₂,
   e) NH₂, oder
   f) N(H)-L
      steht.

Besonders bevorzugte Verbindungen der Erfindung gemäß der Formel (I) zeichnen sich dadurch aus, dass
A für
   a) Hydroxyl,
   b) verzweigtes oder unverzweigtes C₁-C₅ Alkoxy oder
   c) NH₂
      steht.

Bevorzugte Verbindungen der Erfindung gemäß der Formel (I) zeichnen sich dadurch aus, dass
A für
Hydroxyl
steht.

Bevorzugte Verbindungen der Erfindung gemäß der Formel (I) zeichnen sich dadurch aus, dass
A für
NH₂
steht.

Bevorzugte Verbindungen der Erfindung gemäß der Formel (I) zeichnen sich dadurch aus, dass
A für
Ethoxy
steht.

Bevorzugte Verbindungen der Erfindung gemäß der Formel (I) zeichnen sich dadurch aus, dass
G für
   a) Hydroxyl,
   b) verzweigtes oder unverzweigtes O-C₁-C₄ Alkyl, oder
   c) O-C₂H₄-OMe
      steht.

Weitere bevorzugte Verbindungen der Erfindung gemäß der Formel (I) zeichnen sich dadurch aus, dass
G für
a) Hydroxyl, oder
b) verzweigtes oder unverzweigtes O-C₁-C₄ Alkyl
   steht.

Besonders bevorzugte Verbindungen der Erfindung gemäß der Formel (I) zeichnen sich dadurch aus, dass
G für
   a) Hydroxyl,
   b) Methoxy oder
   c) Ethoxy
      steht.

Besonders bevorzugte Verbindungen der Erfindung gemäß der Formel (I) zeichnen sich dadurch aus, dass
G für
Methoxy
steht.

Bevorzugte Verbindungen der Erfindung gemäß der Formel (I) zeichnen sich dadurch aus, dass
R¹ und R² für
   a) Wasserstoff,
   b) verzweigtes oder unverzweigtes ¹⁸F-C₆-C₈ Alkoxy,
   c) verzweigtes oder unverzweigtes ¹⁸F-C₆-C₈ Alkyl,
   d) verzweigtes oder unverzweigtes ¹⁸F-C₆-C₈ Alkenyl,
   e) verzweigtes oder unverzweigtes ¹⁸F-C₆-C₈ Alkinyl, oder
   f) Hydroxyl,
      mit der Maßgabe, dass genau einer der Substituenten R¹ oder R² genau ein ¹⁸F-Isotop beinhaltet und der jeweils andere Substituent Wasserstoff ist,
      steht.

Ein weiterer besonderer Gegenstand der Erfindung sind Verbindungen der allgemeinen Formel I worin
R¹ für
a) verzweigtes oder unverzweigtes ¹⁸F-C₆ Alkoxy,
b) verzweigtes oder unverzweigtes ¹⁸F-C₆ Alkyl,
c) verzweigtes oder unverzweigtes ¹⁸F-C₆ Alkenyl, oder
d) verzweigtes oder unverzweigtes ¹⁸F-C₆ Alkinyl
   steht.

Unverzweigtes ¹⁸F-C₆ Alkoxy ist ¹⁸F-Hexoxy.
Unverzweigtes ¹⁸F-C₆ Alkyl ist ¹⁸F-Hexyl.
Unverzweigtes ¹⁸F-C₆ Alkenyl ist ¹⁸F-Hexenyl.
Unverzweigtes ¹⁸F-C₆ Alkinyl ist ¹⁸F-Hexinyl.

Ein weiterer besonderer Gegenstand der Erfindung sind Verbindungen der allgemeinen Formel I worin
R¹ für ¹⁸F-Hexoxy oder ¹⁸F-Hexyl und R2 für Wasserstoff steht.

Bevorzugte Verbindungen der Erfindung gemäß der Formel (I) zeichnen sich dadurch aus, dass R¹ und R² ausgewählt ist aus der Gruppe Wasserstoff, ¹⁸F-Hexoxy, ¹⁸F-Heptoxy, ¹⁸F-Octoxy, ¹⁸F-Nonoxy, ¹⁸F-Decoxy, ¹⁸F-Hexyl, ¹⁸F-Heptyl, ¹⁸F-Octyl, ¹⁸F-Nonyl, und ¹⁸F-Decyl mit der Maßgabe, dass einer der Substituenten R¹ oder R² genau ein ¹⁸F Isotop beinhaltet und der jeweils andere Substituent Wasserstoff ist.

Bevorzugte Verbindungen der Erfindung gemäß der Formel (I) zeichnen sich dadurch aus, dass
L für
a) Methyl,
b) Ethyl,
c) Propyl,
d) iso-Propyl,
e) -C₂H₄-OMe, oder
f) -C₂H₄-O-C₂H₄-OMe
   steht.

Besonders bevorzugte Verbindungen der Erfindung gemäß der Formel (I) zeichnen sich dadurch aus, dass
L für
a) Methyl oder
b) Ethyl
   steht.

Ebenfalls bevorzugte Verbindungen der Erfindung gemäß der Formel (I) zeichnen sich dadurch aus, dass Z ist ausgewählt aus der Gruppe Na⁺, K⁺, Ca²⁺ und Mg²⁺.
Bevorzugtes Z ist Na⁺.

Alle möglichen Diastereomere und Enantiomere der bevorzugten Verbindungen nach Formel (I) sind Teil des vorliegenden Erfindungsgegenstandes.

Bevorzugte Verbindungen der Erfindung gemäß der Formel (I) zeichnen sich dadurch aus, dass Formel (I) 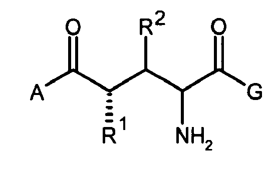 ist.

Das Verfahren zur Herstellung der erfindungsgemäßen Verbindungen der allgemeinen Formel (I) zeichnet sich dadurch aus indem
- eine oder mehrere vorhandene Schutzgruppen einer Verbindung nach Formel (II) abgespalten wird oder werden.

Weiterhin besonders bevorzugt ist jede einzelne der Verbindungen aus der folgenden Gruppe, wobei alle möglichen Diastereomere und Enantiomere Teil des vorliegenden Erfindungsgegenstandes sind
a) 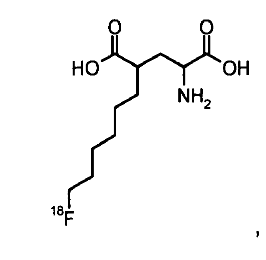 und
b)

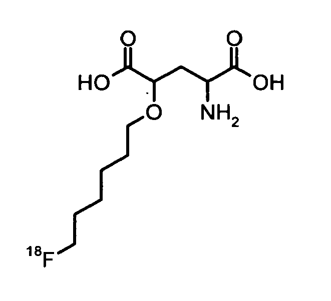

In einem weiteren Aspekt bezieht sich somit die vorliegende Erfindung auf Verbindungen der Formel (II): 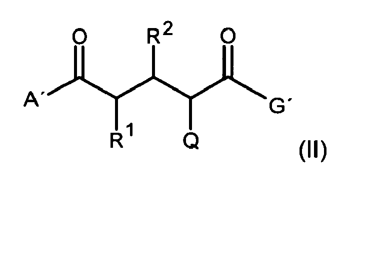 worin,
A' für
   a) Hydroxyl,
   b) verzweigtes oder unverzweigtes C₁-C₅ Alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ Alkyl)ₙ-O-C₁-C₄ Alkyl,
   e) N(C₁-C₅ Alkyl)₂,
   f) NH₂,
   g) N(H)-L', oder
   h) O-L'
      steht,
G' für
   a) hydroxyl,
   b) O-Z',
   c) verzweigtes oder unverzweigtes O-C₁-C₅ Alkyl,
   d) verzweigtes oder unverzweigtes O-C₂-C₅ Alkenyl,
   e) verzweigtes oder unverzweigtes O-C₁-C₅ Alkyl-(O-C₁-C₄ Alkyl)ₙ-O-C₁-C₄ Alkyl,
   f) verzweigtes oder unverzweigtes O-C₂-C₅ Alkinyl oder
   g) Triphenylmethoxy
      steht,
R¹ und R² für
   a) Wasserstoff,
   b) verzweigtes oder unverzweigtes ¹⁸F-C₆-C₁₀ Alkoxy,
   c) verzweigtes oder unverzweigtes ¹⁸F-C₆-C₁₀ Alkyl,
   d) verzweigtes oder unverzweigtes ¹⁸F-C₆-C₁₀ Alkenyl,
   e) verzweigtes oder unverzweigtes ¹⁸F-C₆-C₁₀ Alkinyl,
   f) Hydroxyl,
   g) verzweigtes oder unverzweigtes C₁-C₅ Alkyl oder
   h) verzweigtes oder unverzweigtes C₁-C₅ Alkoxy
      steht,
      mit der Maßgabe, dass genau einer der Substituenten R¹ oder R² genau ein ¹⁸F Isotop und der jeweils andere Substituent kein ¹⁸F Isotop beinhaltet,
Q für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   e) N(H)-2,2,2-Trichlorethoxycarbonyl,
   f) N(H)-1,1-Dimethylpropinyl,
   g) N(H)-1-Methyl-1-phenyl-ethoxycarbonyl,
   h) N(H)-1-Methyl-1-(4-biphenylyl)-ethoxycarbonyl,
   i) N(H)-cyclobutylcarbonyl,
   j) N(H)-1-Methylcyclobutylcarbonyl,
   k) N(H)-Vinylcarbonyl,
   l) N(H)-Allylcarbonyl,
   m) N(H)-Adamantylcarbonyl,
   n) N(H)-Diphenylmethylcarbonyl,
   o) N(H)-Cinnamylcarbonyl,
   p) N(H)-Formyl,
   q) N(H)-Benzoyl,
   r) N(H)-Trityl,
   s) N(H)-p-Methoxyphenyl-diphenylmethyl,
   t) N(H)-di-(p-Methoxyphenyl)-phenylmethyl,
   u) 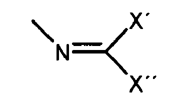 oder
   v) N-(tert-Butoxycarbonyl)₂,
      steht,
L' für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl, oder
   d) verzweigtes oder unverzweigtes C₂-C₅ Alkinyl
      steht,
X' und X'' unabhängig voneinander für
   a) verzweigtes oder unverzweigtes C₁-C₅ Alkyl,
   b) substituiertes oder unsubstituiertes Aryl,
   c) substituiertes oder unsubstituiertes Aralkyl, oder
   d) substituiertes oder unsubstituiertes heteroaryl
      steht,
Z' für ein Metallkationenequivalent
   steht,
wobei n = 0, 1, 2 oder 3 ist und alle möglichen Diastereomere und Enantiomere Teil des vorliegenden Erfindungsgegenstandes sind.

Bevorzugte Verbindungen der Erfindung gemäß der Formel (II) zeichnen sich dadurch aus, dass
A' für
a) Hydroxyl,
b) Methoxy,
c) Ethoxy,
d) Propoxy,
e) NMe₂,
f) NEt₂,
g) NH₂,
h) N(H)-L,
i) O-L, oder
j) O-Z
   steht.

Weitere bevorzugte Verbindungen der Erfindung gemäß der Formel (II) zeichnen sich dadurch aus, dass
A' für
a) Hydroxyl,
b) Methoxy,
c) Ethoxy,
d) NMe₂,
e) NH₂, oder
f) N(H)-L
   steht.

Besonders bevorzugte Verbindungen der Erfindung gemäß der Formel (II) zeichnen sich dadurch aus, dass
A' für
a) Hydroxyl,
b) verzweigtes oder unverzweigtes C₁-C₅ Alkoxy oder
c) NH₂
   steht.

Bevorzugte Verbindungen der Erfindung gemäß der Formel (II) zeichnen sich dadurch aus, dass
A' für
Hydroxyl
steht.

Bevorzugte Verbindungen der Erfindung gemäß der Formel (II) zeichnen sich dadurch aus, dass
A'für
NH₂
steht.

Bevorzugte Verbindungen der Erfindung gemäß der Formel (II) zeichnen sich dadurch aus, dass
A' für
Ethoxy
steht.

Bevorzugte Verbindungen der Erfindung gemäß der Formel (II) zeichnen sich dadurch aus, dass
G' für
a) Hydroxyl,
b) verzweigtes oder unverzweigtes O-C₁-C₄ Alkyl, oder
c) O-C₂H₄-OMe
   steht.

Weitere bevorzugte Verbindungen der Erfindung gemäß der Formel (II) zeichnen sich dadurch aus, dass
G' für
a) Hydroxyl, oder
b) verzweigtes oder unverzweigtes O-C₁-C₄ Alkyl
   steht.

Besonders bevorzugte Verbindungen der Erfindung gemäß der Formel (II) zeichnen sich dadurch aus, dass
G' für
a) Hydroxyl,
b) Methoxy oder
c) Ethoxy
   steht.

Besonders bevorzugte Verbindungen der Erfindung gemäß der Formel (II) zeichnen sich dadurch aus, dass
G' für
Methoxy
steht.

Bevorzugte Verbindungen der Erfindung gemäß der Formel (II) zeichnen sich dadurch aus, dass
R¹ und R² für
a) Wasserstoff,
b) verzweigtes oder unverzweigtes ¹⁸F-C₆-C₈ Alkoxy,
c) verzweigtes oder unverzweigtes ¹⁸F-C₆-C₈ Alkyl,
d) verzweigtes oder unverzweigtes ¹⁸F-C₆-C₈ Alkenyl, oder
e) verzweigtes oder unverzweigtes ¹⁸F-C₆-C₈ Alkinyl,
   mit der Maßgabe, dass genau einer der Substituenten R¹ oder R² genau ein ¹⁸F-Isotop beinhaltet und der jeweils andere Substituent Wasserstoff ist,
   steht.

Ein weiterer besonderer Gegenstand der Erfindung sind Verbindungen der allgemeinen Formel I worin
R¹ für
a) verzweigtes oder unverzweigtes ¹⁸F-C₆ Alkoxy,
b) verzweigtes oder unverzweigtes ¹⁸F-C₆ Alkyl,
c) verzweigtes oder unverzweigtes ¹⁸F-C₆ Alkenyl, oder
d) verzweigtes oder unverzweigtes ¹⁸F-C₆ Alkinyl
   steht.

Unverzweigtes ¹⁸F-C₆ Alkoxy ist ¹⁸F-Hexoxy.
Unverzweigtes ¹⁸F-C₆ Alkyl ist ¹⁸F-Hexyl.
Unverzweigtes ¹⁸F-C₆ Alkenyl ist ¹⁸F-Hexenyl.
Unverzweigtes ¹⁸F-C₆ Alkinyl ist ¹⁸F-Hexinyl.

Ein weiterer besonderer Gegenstand der Erfindung sind Verbindungen der allgemeinen Formel I worin
R¹ für ¹⁸F-Hexoxy oder ¹⁸F-Hexyl und R2 für Wasserstoff steht.

Bevorzugte Verbindungen der Erfindung gemäß der Formel (I) zeichnen sich dadurch aus, dass R¹ und R² ausgewählt ist aus der Gruppe Wasserstoff, ¹⁸F-Hexoxy, ¹⁸F-Heptoxy, ¹⁸F-Octoxy, ¹⁸F-Nonoxy, ¹⁸F-Decoxy, ¹⁸F-Hexyl, ¹⁸F-Heptyl, ¹⁸F-Octyl, ¹⁸F-Nonyl, und ¹⁸F-Decyl mit der Maßgabe, dass einer der Substituenten R¹ oder R² genau ein ¹⁸F Isotop beinhaltet und der jeweils andere Substituent Wasserstoff ist.

Bevorzugte Verbindungen der Erfindung gemäß der Formel (II) zeichnen sich dadurch aus, dass
L' für
a) Methyl,
b) Ethyl,
c) Propyl,
d) iso-Propyl,
e) -C₂H₄-OMe, oder
f) -C₂H₄-O-C₂H₄-OMe
   steht.

Besonders bevorzugte Verbindungen der Erfindung gemäß der Formel (II) zeichnen sich dadurch aus, dass
L' für
a) Methyl oder
b) Ethyl
   steht.

Ebenfalls bevorzugte Verbindungen der Erfindung gemäß der Formel (II) zeichnen sich dadurch aus, dass Z' ist ausgewählt aus der Gruppe Na⁺, K⁺, Ca²⁺ und Mg²⁺.
Bevorzugtes Z' ist Na⁺.

Alle möglichen Diastereomere und Enantiomere der bevorzugten Verbindungen nach Formel (II) sind Teil des vorliegenden Erfindungsgegenstandes.

Bevorzugte Verbindungen der Erfindung gemäß der Formel (II) zeichnen sich dadurch aus, dass Formel (II)

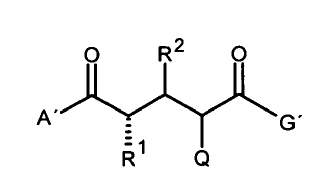

Bevorzugte Verbindungen der Erfindung gemäß der Formel (II) zeichnen sich dadurch aus, dass
Q für
a) N(H)-tert-Butoxycarbonyl,
b) N(H)-Benzyloxycarbonyl,
c) N-(tert-Butoxycarbonyl)₂, oder
d) 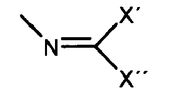 steht.

Weitere bevorzugte Verbindungen der Erfindung gemäß der Formel (II) zeichnen sich dadurch aus, dass
Q für
a) N(H)-tert-Butoxycarbonyl,
b) N-(tert-Butoxycarbonyl)₂, oder
c) 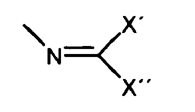 steht.

Besonders bevorzugte Verbindungen der Erfindung gemäß der Formel (II) zeichnen sich dadurch aus, dass
Q für
a) N(H)-tert-Butoxycarbonyl, oder
b) 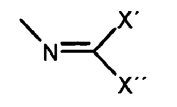 steht.

Besonders bevorzugte Verbindungen der Erfindung gemäß der Formel (II) zeichnen sich dadurch aus, dass
Q für N(H)-tert-Butoxycarbonyl steht.

Bevorzugte Verbindungen der Erfindung gemäß der Formel (II) zeichnen sich dadurch aus, dass
L' für
a) Methyl,
b) Ethyl,
c) Propyl,
d) iso-Propyl,
e) -C₂H₄-OMe, oder
f) -C₂H₄-O-C₂H₄-OMe
   steht.

Bevorzugte Verbindungen der Erfindung gemäß der Formel (II) zeichnen sich dadurch aus, dass
X' und X'' unabhängig voneinander für
a) verzweigtes oder unverzweigtes C₁-C₅ Alkyl,
b) substituiertes oder unsubstituiertes Aryl oder
c) substituiertes oder unsubstituiertes Aralkyl
   steht.

Weitere bevorzugte Verbindungen der Erfindung gemäß der Formel (II) zeichnen sich dadurch aus, dass
X' und X'' unabhängig voneinander für
a) verzweigtes oder unverzweigtes C₁-C₅ Alkyl, oder
b) substituiertes oder unsubstituiertes Aryl
   steht.

Besonders bevorzugte Verbindungen der Erfindung gemäß der Formel (II) zeichnen sich dadurch aus, dass
X' und X'' für Phenyl bzw. für in 2-Position substituiertes Phenyl
steht.

Wobei alle möglichen Diastereomere und Enantiomere der bevorzugten Verbindungen der Erfindung gemäß Formel (II) Teil des vorliegenden Erfindungsgegenstandes sind.

Weiterhin besonders bevorzugt ist jede einzelne der Verbindungen aus der folgenden Gruppe, wobei alle möglichen Diastereomere und Enantiomere Teil des vorliegenden Erfindungsgegenstandes sind 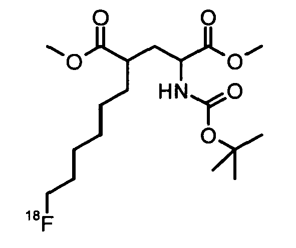 und

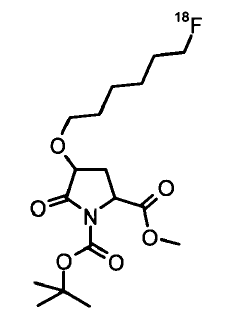

Das Verfahren zur Herstellung der erfindungsgemäßen Verbindungen der allgemeinen Formel (II) zeichnet sich dadurch aus, dass die Mehrzahl der Verbindung nach Formel (II), aus einer Vorläuferverbindung der Verbindung der Formel (III) nach Einführung des ¹⁸F lsotops entstehen kann.

In einem dritten Aspekt bezieht sich die vorliegende Erfindung auf Verbindungen der Formel (III): 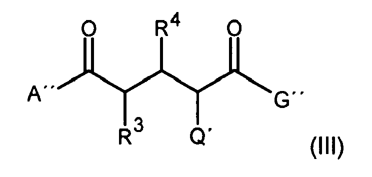 worin
A'' für
   a) verzweigtes oder unverzweigtes C₁-C₅ Alkoxy,
   b) verzweigtes oder unverzweigtes Hydroxy C₁-C₅ Alkoxy,
   c) verzweigtes oder unverzweigtes O-C₁-C₅ Alkyl-(O-C₁-C₄ alkyl)ₙ-O-C₁-C₄ alkyl,
   d) N(C₁-C₅ Alkyl)₂,
   e) NH₂,
   f) N(H)-L'' oder
   g) O-L''
      steht,
G'' für
   a) O-Z'',
   b) verzweigtes oder unverzweigtes O-C₁-C₅ Alkyl,
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl,
   e) verzweigtes oder unverzweigtes O-C₂-C₅ Alkinyl, oder
   f) Triphenylmethoxy
      steht,
R³ und R⁴ für
   a) Wasserstoff,
   b) verzweigtes oder unverzweigtes E-C₆-C₁₀ Alkoxy,
   c) verzweigtes oder unverzweigtes E-C₆-C₁₀ Alkyl,
   d) verzweigtes oder unverzweigtes E-C₆-C₁₀ Alkenyl,
   e) verzweigtes oder unverzweigtes E-C₆-C₁₀ Alkinyl,
   f) Hydroxyl,
   g) verzweigtes oder unverzweigtes C₁-C₅ Alkyl oder
   h) verzweigtes oder unverzweigtes C₁-C₅ Alkoxy
      steht, mit der Maßgabe, dass genau einer der Substituenten R³ oder R⁴ ein E beinhaltet und der jeweils andere Substituent kein E beinhaltet,
E für
   a) Chloro,
   b) Bromo,
   c) Methansulfonyloxy,
   d) Trifluormethansulfonyloxy,
   e) Nonafluorbutyloxy,
   f) Tosyloxy, oder
   g) lodo
      steht,
Q' für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   g) N(H)-2,2,2-Trichlorethoxycarbonyl,
   h) N(H)-1,1-Dimethylpropinyl,
   i) N(H)-1-Methyl-1-phenyl-ethoxycarbonyl,
   j) N(H)-1-Methyl-1-(4-biphenylyl)-ethoxycarbonyl,
   k) N(H)-Cyclobutylcarbonyl,
   l) N(H)-1-Methylcyclobutylcarbonyl,
   m) N(H)-Vinylcarbonyl,
   n) N(H)-Allylcarbonyl,
   o) N(H)-Adamantylcarbonyl,
   p) N(H)-Diphenylmethylcarbonyl,
   q) N(H)-Cinnamylcarbonyl,
   r) N(H)-Formyl,
   s) N(H)-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 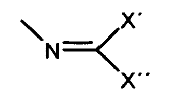 oder
   x) N-(tert-Butoxycarbonyl)₂,
      steht,
L" für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl oder
   d) verzweigtes oder unverzweigtes C₂-C₅ Alkinyl
      steht,
X' and X'' unabhängig voneinander für
   a) verzweigtes oder unverzweigtes C₁-C₅ Alkyl,
   b) substituiertes oder unsubstituiertes Aryl
   c) substituiertes oder unsubstituiertes Alkylaryl oder
   d) substituiertes oder unsubstituiertes Heteroaryl
      steht, und
Z'' für ein Metallkationenequivalent steht,
   wobei n = 0, 1, 2 oder 3 ist und
   alle möglichen Diastereomere und Enantiomere Teil des vorliegenden Erfindungsgegenstandes sind.

Bevorzugte Verbindungen der Erfindung gemäß der Formel (I) zeichnen sich dadurch aus, dass
A für
a) verzweigtes oder unverzweigtes C₁-C₅ Alkoxy, oder
b) NH₂,
   steht.

Weitere bevorzugte Verbindungen der Erfindung gemäß der Formel (I) zeichnen sich dadurch aus, dass
A für
a) Methoxy,
b) Ethoxy, oder
c) NH₂,
   steht.

Bevorzugte Verbindungen der Erfindung gemäß der Formel (I) zeichnen sich dadurch aus, dass
A'' für
Methoxy
steht.

Bevorzugte Verbindungen der Erfindung gemäß der Formel (I) zeichnen sich dadurch aus, dass
A'' für
NH₂
steht.

Bevorzugte Verbindungen der Erfindung gemäß der Formel (I) zeichnen sich dadurch aus, dass
A'' für
Ethoxy
steht.

Bevorzugte Verbindungen der Erfindung gemäß der Formel (III) zeichnen sich dadurch aus, dass
G'' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl, oder
e) Triphenylmethoxy
   steht.

Weiter bevorzugte Verbindungen der Erfindung gemäß der Formel (III) zeichnen sich dadurch aus, dass
G'' für
a) Methoxy,
b) Ethoxy,
c) tert-Butoxy
   steht.

Besonders bevorzugte Verbindungen der Erfindung gemäß der Formel (III) zeichnen sich dadurch aus, dass
G'' für
a) Methoxy
   steht.

Bevorzugte Verbindungen der Erfindung gemäß der Formel (III) zeichnen sich dadurch aus, dass
R³ und R⁴ für
a) Wasserstoff,
b) verzweigtes oder unverzweigtes E-C₆-C₁₀ Alkoxy,
c) verzweigtes oder unverzweigtes E-C₆-C₁₀ Alkyl,
d) verzweigtes oder unverzweigtes E-C₆-C₁₀ Alkenyl, oder
e) verzweigtes oder unverzweigtes E-C₆-C₁₀ Alkinyl,
   steht, mit der Maßgabe, dass genau einer der Substituenten R³ oder R⁴ ein E beinhaltet und der jeweils andere Substituent Wasserstoff ist.

Bevorzugte Verbindungen der Erfindung gemäß der Formel (III) zeichnen sich dadurch aus, dass
R³ und R⁴ für
   a) Wasserstoff,
   b) verzweigtes oder unverzweigtes E-C₆-C₈ Alkoxy,
   c) verzweigtes oder unverzweigtes E-C₆-C₈ Alkyl,
   d) verzweigtes oder unverzweigtes E-C₆-C₈ Alkenyl, oder
   e) verzweigtes oder unverzweigtes E-C₆-C₈ Alkinyl,
   f) Hydroxyl,
   g) verzweigtes oder unverzweigtes C₁-C₅ Alkyl oder
   h) verzweigtes oder unverzweigtes C₁-C₅ Alkoxy
      steht,
      steht, mit der Maßgabe, dass genau einer der Substituenten R³ oder R⁴ ein E beinhaltet und der jeweils andere Substituent kein E beinhaltet,

Ein weiterer besonderer Gegenstand der Erfindung sind Verbindungen der allgemeinen Formel **III** worin
R³ für
a) verzweigtes oder unverzweigtes E-C₆ Alkoxy,
b) verzweigtes oder unverzweigtes E-C₆ Alkyl,
c) verzweigtes oder unverzweigtes E-C₆ Alkenyl, oder
d) verzweigtes oder unverzweigtes E-C₆ Alkinyl
   steht.

Unverzweigtes E-C₆ Alkoxy ist E-Hexoxy.
Unverzweigtes E-C₆ Alkyl ist E-Hexyl.
Unverzweigtes E-C₆ Alkenyl ist E-Hexenyl.
Unverzweigtes E-C₆ Alkinyl ist E-Hexinyl.

Ein weiterer besonderer Gegenstand der Erfindung sind Verbindungen der allgemeinen Formel **III** worin
R³ für E-Hexoxy oder E-Hexyl und R⁴ für Wasserstoff steht.

Bevorzugte Verbindungen der Erfindung gemäß der Formel (III) zeichnen sich dadurch aus, dass
E für
a) Chloro,
b) Bromo,
c) Methansulfonyloxy,
d) Trifluormethansulfonyloxy,
e) Tosyloxy oder
f) lodo
   steht.

Weiter bevorzugte Verbindungen der Erfindung gemäß der Formel (III) zeichnen sich dadurch aus, dass
E für
a) Chloro,
b) Bromo,
c) Methansulfonyloxy,
d) Trifluormethansulfonyloxy,
e) Tosyloxy oder
f) lodo
   steht.

Besonders bevorzugte Verbindungen der Erfindung gemäß der Formel (III) zeichnen sich dadurch aus, dass
E für
a) Bromo, oder
b) Methansulfonyloxy,
   steht.

Bevorzugte Verbindungen der Erfindung gemäß der Formel (III) zeichnen sich dadurch aus, dass
Q' für
a) N(H)-tert-Butoxycarbonyl,
b) N(H)-Benzyloxycarbonyl,
c) N(H)-Trityl, oder
d) 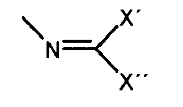 steht.

Weiter bevorzugte Verbindungen der Erfindung gemäß der Formel (III) zeichnen sich dadurch aus, dass
Q' für
a) N(H)-tert-Butoxycarbonyl, oder
b) 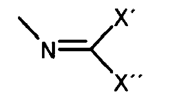 steht.

Bevorzugte Verbindungen der Erfindung gemäß der Formel (III) zeichnen sich dadurch aus, dass
L" für
a) verzweigtes oder unverzweigtes C₁-C₅ Alkyl,
b) verzweigtes oder unverzweigtes C₂-C₅ Alkenyl, oder
c) verzweigtes oder unverzweigtes C₂-C₅ Alkinyl

Bevorzugte Verbindungen der Erfindung gemäß der Formel (III) zeichnen sich dadurch aus, dass
L" für
a) Methyl,
b) Ethyl,
c) Propyl,
d) iso-Propyl,
e) -C₂H₄-OMe, oder
f) -C₂H₄-O-C₂H₄-OMe
   steht.

Weiter bevorzugte Verbindungen der Erfindung gemäß der Formel (III) zeichnen sich dadurch aus, dass
L" für
a) Methyl, oder
b) Ethyl
   steht.

Bevorzugte Verbindungen der Erfindung gemäß der Formel (III) zeichnen sich dadurch aus, dass
X' and X'' unabhängig voneinander für
a) verzweigtes oder unverzweigtes C₁-C₅ Alkyl,
b) substituiertes oder unsubstituiertes Aryl, oder
c) Aralkyl
   steht.

Weiter bevorzugte Verbindungen der Erfindung gemäß der Formel (III) zeichnen sich dadurch aus, dass
X' und X'' unabhängig voneinander für
a) verzweigtes oder unverzweigtes C₁-C₅ Alkyl, und
b) substituiertes oder unsubstituiertes Aryl
   steht.

Besonders bevorzugte Verbindungen der Erfindung gemäß der Formel (III) zeichnen sich dadurch aus, dass
X' und X'' für Phenyl bzw. für in 2-Position substituiertes Phenyl steht.

Wobei alle möglichen Diastereomere und Enantiomere der bevorzugten Verbindungen gemäß Formel (III) Teil des vorliegenden Erfindungsgegenstandes sind.

Alle möglichen Diastereomere und Enantiomere der bevorzugten Verbindungen nach Formel (II) sind Teil des vorliegenden Erfindungsgegenstandes.
Bevorzugte Verbindungen der Erfindung gemäß der Formel (III) zeichnen sich dadurch aus, dass Formel (III)

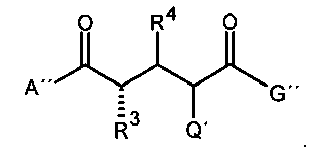

Ebenfalls bevorzugte Verbindungen der Erfindung gemäß der Formel (III) zeichnen sich dadurch aus, dass Z' ist ausgewählt aus der Gruppe Na⁺, K⁺, Ca²⁺ und Mg²⁺.
Bevorzugtes Z' ist Na⁺.

In einem weiteren Aspekt bezieht sich die vorliegende Erfindung auf Verbindungen der Formel (IV): 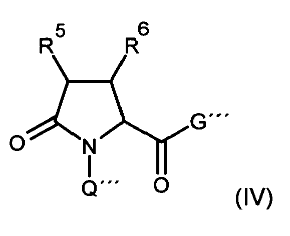 worin
G''' für
   a) verzweigtes oder unverzweigtes O-C₁-C₅ Alkyl,
   b) verzweigtes oder unverzweigtes O-C₂-C₅ Alkenyl,
   c) verzweigtes oder unverzweigtes O-C₁-C₅ Alkyl-(O-C₁-C₄ alkyl)ₙ-O-C₁-C₄ alkyl,
   d) verzweigtes oder unverzweigtes O-C₂-C₅ Alkinyl, oder
   e) triphenylmethoxy
      steht,
R⁵ und R⁶ für
   a) Wasserstoff,
   b) Hydroxyl,
   c) verzweigtes oder unverzweigtes C₁-C₅ Alkyl,
   d) verzweigtes oder unverzweigtes C₁-C₅ Alkoxy oder
   e) R⁷-E'
      steht, mit der Maßgabe, dass genau einer der Substituenten R⁵ oder R⁶ ein E' beinhaltet und der jeweils andere Substituent kein E' beinhaltet,

E' für
   a) Chloro,
   b) Bromo,
   c) Methansulfonyloxy,
   d) Trifluormethansulfonyloxy,
   e) Nonafluorbutyloxy,
   f) Tosyloxy oder
   g) lodo
      steht,
R⁷ für
   a) verzweigtes oder unverzweigtes C₆-C₁₀ Alkoxy,
   b) verzweigtes oder unverzweigtes C₆-C₁₀ Alkyl,
   c) verzweigtes oder unverzweigtes C₆-C₁₀ Alkenyl, oder
   d) verzweigtes oder unverzweigtes C₆-C₁₀ Alkinyl
      steht,
Q''' für
   a) N-tert-Butoxycarbonyl,
   b) N-Allyloxycarbonyl,
   c) N-Benzyloxycarbonyl,
   d) N-Ethoxycarbonyl,
   e) N-Methoxycarbonyl,
   f) N-Propoxycarbonyl,
   g) N-2,2,2-Trichlorethoxycarbonyl,
   h) Wasserstoff,
   i) N-1-Methyl-1-phenyl-ethoxycarbonyl,
   j) N-1-Methyl-1-(4-biphenylyl)-ethoxycarbonyl,
   k) N-cyclobutylcarbonyl,
   l) N-1-Methylcyclobutylcarbonyl,
   m) N-Vinylcarbonyl,
   n) N-Allylcarbonyl,
   o) N-Adamantylcarbonyl,
   p) N-Diphenylmethylcarbonyl,
   q) N-Cinnamylcarbonyl,
   r) N-Formyl, oder
   s) N-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 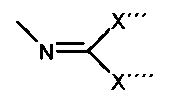 oder
   x) N-(tert-Butoxycarbonyl)₂,
      steht,
X''' and X'''' unabhängig voneinander für
   a) verzweigtes oder unverzweigtes C₁-C₅ Alkyl,
   b) substituiertes oder unsubstituiertes Aryl,
   c) substituiertes oder unsubstituiertes Alkylaryl, oder
   d) substituiertes oder unsubstituiertes Heteroaryl
      steht,
   wobei n = 0, 1, 2 oder 3 ist und
   alle Diastereomere und Enantiomere umfasst sind.

Besonders bevorzugte Verbindungen der Erfindung gemäß der Formel (IV) zeichnen sich dadurch aus, dass
G''' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl, oder
e) Triphenylmethoxy
   steht.

Weiter bevorzugte Verbindungen der Erfindung gemäß der Formel (IV) zeichnen sich dadurch aus, dass
G''' für
   a) Methoxy,
   b) Ethoxy, oder
   c) tert-Butoxy
      steht.

Besonders bevorzugte Verbindungen der Erfindung gemäß der Formel (IV) zeichnen sich dadurch aus, dass
G''' für
a) Methoxy
   steht.

Bevorzugte Verbindungen der Erfindung gemäß der Formel (IV) zeichnen sich dadurch aus, dass
E' für
a) Chloro,
b) Bromo,
c) Methansulfonyloxy,
d) Trifluormethansulfonyloxy,
e) Tosyloxy oder
f) lodo
   steht.

Weiter bevorzugte Verbindungen der Erfindung gemäß der Formel (IV) zeichnen sich dadurch aus, dass
E' für
a) Bromo,
b) Methansulfonyloxy,
c) Trifluormethansulfonyloxy,
d) Tosyloxy oder
e) lodo
   steht.

Besonders bevorzugte Verbindungen der Erfindung gemäß der Formel (IV) zeichnen sich dadurch aus, dass
E' für
a) Bromo, oder
b) Methansulfonyloxy
   steht.

Bevorzugte Verbindungen der Erfindung gemäß der Formel (IV) zeichnen sich dadurch aus, dass
Q' für
a) N(H)-tert-Butoxycarbonyl,
b) N(H)-Benzyloxycarbonyl,
c) N(H)-Trityl, oder
d) 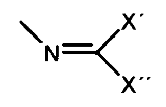 steht.

Weiter bevorzugte Verbindungen der Erfindung gemäß der Formel (IV) zeichnen sich dadurch aus, dass
Q' für
a) N(H)-tert-Butoxycarbonyl, oder
b) 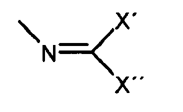 steht.

Bevorzugte Verbindungen der Erfindung gemäß der Formel (III) zeichnen sich dadurch aus, dass
X' and X'' unabhängig voneinander für
a) verzweigtes oder unverzweigtes C₁-C₅ Alkyl,
b) substituiertes oder unsubstituiertes Aryl, oder
c) substituiertes oder unsubstituiertes Aralkyl
   steht.

Weiter bevorzugte Verbindungen der Erfindung gemäß der Formel (III) zeichnen sich dadurch aus, dass
X' und X'' unabhängig voneinander für
   a) verzweigtes oder unverzweigtes C₁-C₅ Alkyl, und
   b) substituiertes oder unsubstituiertes Aryl
      steht.

Besonders bevorzugte Verbindungen der Erfindung gemäß der Formel (III) zeichnen sich dadurch aus, dass
X' und X'' für Phenyl bzw. für in 2-Position substituiertes Phenyl steht.

Alle möglichen Diastereomere und Enantiomere der bevorzugten Verbindungen nach Formel (II) sind Teil des vorliegenden Erfindungsgegenstandes.

Bevorzugte Verbindungen der Erfindung gemäß der Formel (IV) zeichnen sich dadurch aus, dass Formel (IV)

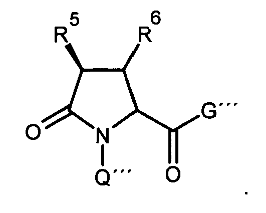

Ebenfalls bevorzugte Verbindungen der Erfindung gemäß der Formel (IV) zeichnen sich dadurch aus, dass Z' ist ausgewählt aus der Gruppe Na⁺, K⁺, Ca²⁺ und Mg²⁺.
Bevorzugtes Z' ist Na⁺.

In einem weiteren Aspekt bezieht sich die vorliegende Erfindung auf Verwendung von Verbindungen der Formel (IV) zur Herstellung von Verbindungen der Formel (I) oder (II): 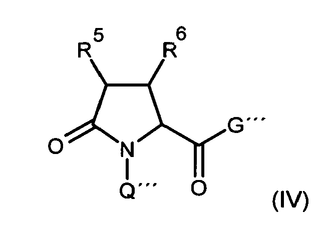 worin
G''' für
   a) verzweigtes oder unverzweigtes O-C₁-C₅ Alkyl,
   b) verzweigtes oder unverzweigtes O-C₂-C₅ Alkenyl,
   c) verzweigtes oder unverzweigtes O-C₁-C₅ Alkyl-(O-C₁-C₄ alkyl)ₙ-O-C₁-C₄ alkyl,
   d) verzweigtes oder unverzweigtes O-C₂-C₅ Alkinyl, oder
   e) Triphenylmethoxy
      steht,
R⁵ und R⁶ für
   a) Wasserstoff,
   b) Hydroxyl,
   c) verzweigtes oder unverzweigtes C₁-C₅ Alkyl,
   d) verzweigtes oder unverzweigtes C₁-C₅ Alkoxy oder
   e) R⁷-E'
      steht, mit der Maßgabe, dass genau einer der Substituenten R⁵ oder R⁶ ein E' beinhaltet und der jeweils andere Substituent kein E' beinhaltet,
E' für
   a) Chloro,
   b) Bromo,
   c) Methansulfonyloxy,
   d) Trifluormethansulfonyloxy,
   e) Nonafluorbutyloxy,
   f) Tosyloxy oder
   g) lodo
      steht,
R⁷ für
   a) verzweigtes oder unverzweigtes C₆-C₁₀ Alkoxy,
   b) verzweigtes oder unverzweigtes C₆-C₁₀ Alkyl,
   c) verzweigtes oder unverzweigtes C₆-C₁₀ Alkenyl, oder
   d) verzweigtes oder unverzweigtes C₆-C₁₀ Alkinyl
      steht,
Q''' für
   a) N-tert-Butoxycarbonyl,
   b) N-Allyloxycarbonyl,
   c) N-Benzyloxycarbonyl,
   d) N-Ethoxycarbonyl,
   e) N-Methoxycarbonyl,
   f) N-Propoxycarbonyl,
   g) N-2,2,2-Trichlorethoxycarbonyl,
   h) Wasserstoff,
   i) N-1-Methyl-1-phenyl-ethoxycarbonyl,
   j) N-1-Methyl-1-(4-biphenylyl)-ethoxycarbonyl,
   k) N-cyclobutylcarbonyl,
   l) N-1-Methylcyclobutylcarbonyl,
   m) N-Vinylcarbonyl,
   n) N-Allylcarbonyl,
   o) N-Adamantylcarbonyl,
   p) N-Diphenylmethylcarbonyl,
   q) N-Cinnamylcarbonyl,
   r) N-Formyl, oder
   s) N-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 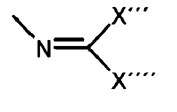 oder
   x) N-(tert-Butoxycarbonyl)₂,
      steht,
X''' and X'''' unabhängig voneinander für
   a) verzweigtes oder unverzweigtes C₁-C₅ Alkyl,
   b) substituiertes oder unsubstituiertes Aryl,
   c) substituiertes oder unsubstituiertes Alkylaryl, oder
   d) substituiertes oder unsubstituiertes Heteroaryl
      steht,
   wobei n = 0, 1, 2 oder 3 ist und
   alle Diastereomere und Enantiomere umfasst sind.

In einem weiteren Aspekt bezieht sich die vorliegende Erfindung auf Bildgebungs-kit enthaltend Verbindungen der allgemeinen Formel III oder IV.

In einem weiteren Aspekt bezieht sich die vorliegende Erfindung auf Pharmazeutische Zusammensetzung enthaltend Verbindungen der allgemeinen Formel I, II, III oder IV und geeignete pharmazeutische Träger Substanzen.

In einem weiteren Aspekt bezieht sich die vorliegende Erfindung auf Verbindungen der allgemeinen Formel V 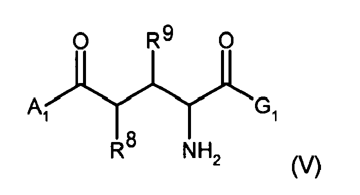 worin
A₁ für
   a) Hydroxyl
   b) verzweigtes oder unverzweigtes C₁-C₅ Alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ Alkyl)ₙ-O-C₁-C₄ Alkyl,
   e) N(C₁-C₅ Alkyl)₂,
   f) NH₂,
   g) N(H)-L₁,
   h) O-L₁ oder
   i) O-Z₁
      steht,
G₁ für
   a) Hydroxyl
   b) O-Z₁
   c) verzweigtes oder unverzweigtes O-C₁-C₅ Alkyl,
   c) verzweigtes oder unverzweigtes O-C₂-C₅ Alkenyl,
   e) verzweigtes oder unverzweigtes O-C₁-C₅ Alkyl-(O-C₁-C₄ Alkyl)ₙ-O-C₁-C₄ Alkyl,
   f) verzweigtes oder unverzweigtes O-C₂-C₅ Alkinyl, oder
   g) Triphenylmethoxy
      steht,
R⁸ und R⁹ für
   a) Wasserstoff,
   b) substituiertes oder unsubstituiertes ¹⁸F-C₆-C₁₀ mono- oder bicyclisches Aryl,
   c) substituiertes oder unsubstituiertes ¹⁸F-C₅-C₁₀ mono- oder bicyclisches Heteroaryl,
   d) substituiertes oder unsubstituiertes ¹⁸F-C₃-C₆ Cyclo-Alkyl,
   e) Hydroxyl,
   f) verzweigtes oder unverzweigtes C₁-C₅ Alkyl oder
   g) verzweigtes oder unverzweigtes C₁-C₅ Alkoxy
      steht,
      mit der Maßgabe, dass einer der Substituenten R³ oder R⁹ genau ein ¹⁸F Isotop beinhaltet und der jeweils andere Substituent kein ¹⁸F Isotop beinhaltet,
L₁ für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ Alkyl)ₙ-O-C₁-C₄ Alkyl oder
   d) verzweigtes oder unverzweigtes C₂-C₅ Alkinyl,
      steht,
Z₁ für ein Metallkationenequivalent steht,
wobei
n = 0, 1, 2 oder 3 ist und
alle Diastereomere und Enantiomere umfasst sind.

Weiterhin besonders bevorzugt ist jede einzelne der Verbindungen aus der folgenden Gruppe, wobei alle möglichen Diastereomere und Enantiomere Teil des vorliegenden Erfindungsgegenstandes sind
a), 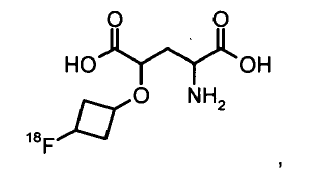
b) 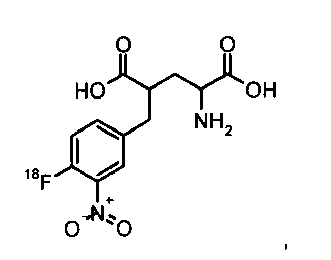
c)

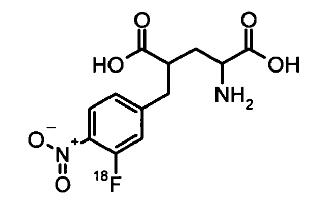

Verbindungen nach Formel V für die Verwendung als Arzneimittel.
Verbindungen nach Formel V für Verwendung für die Bildgebung bei Tumorerkrankungen. Werwendung von Verbindungen nach Formel V zur Herstellung eines Arzneimittels für die Bildgebung bei Tumorerkrankungen.

Verfahren zur Herstellung von Verbindungen der allgemeinen Formel (V) indem
eine oder mehrere vorhandene Schutzgruppen einer Verbindung nach Formel (VI) abgespalten werden.

In einem weiteren Aspekt bezieht sich die vorliegende Erfindung auf Verbindungen der allgemeinen Formel (VI) 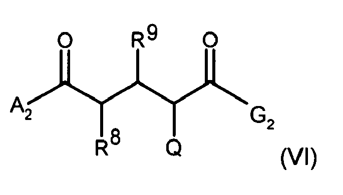 worin,
A₂ für
   a) Hydroxyl,
   b) verzweigtes oder unverzweigtes C₁-C₅ Alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ Alkyl)ₙ-O-C₁-C₄ Alkyl,
   e) N(C₁-C₅ Alkyl)₂,
   f) NH₂,
   g) N(H)-L₂, oder
   h) O-L₂
      steht,
G₂ für
   a) hydroxyl,
   b) O-Z₂,
   c) verzweigtes oder unverzweigtes O-C₁-C₅ Alkyl,
   d) verzweigtes oder unverzweigtes O-C₂-C₅ Alkenyl,
   e) verzweigtes oder unverzweigtes O-C₁-C₅ Alkyl-(O-C₁-C₄ Alkyl)ₙ-O-C₁-C₄ Alkyl,
   f) verzweigtes oder unverzweigtes O-C₂-C₅ Alkinyl oder
   g) Triphenylmethoxy
      steht,
R₈ und R₉ für
   a) Wasserstoff,
   b) substituiertes oder unsubstituiertes ¹⁸F-C₆-C₁₀ mono- oder bicyclisches Aryl,
   c) substituiertes oder unsubstituiertes ¹⁸F-C₅-C₁₀ mono- oder bicyclisches Heteroaryl,
   d) substituiertes oder unsubstituiertes ¹⁸F-C₃-C₆ Cyclo-Alkyl,
   e) Hydroxyl,
   f) verzweigtes oder unverzweigtes C₁-C₅ Alkyl oder
   g) verzweigtes oder unverzweigtes C₁-C₅ Alkoxy
      steht,
      mit der Maßgabe, dass genau einer der Substituenten R₈ oder R⁹ genau ein ¹⁸F Isotop und der jeweils andere Substituent kein ¹⁸F Isotop beinhaltet,
Q₁ für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   e) N(H)-2,2,2-Trichlorethoxycarbonyl,
   f) N(H)-1,1-Dimethylpropinyl,
   g) N(H)-1-Methyl-1-phenyl-ethoxycarbonyl,
   h) N(H)-1-Methyl-1-(4-biphenylyl)-ethoxycarbonyl,
   i) N(H)-cyclobutylcarbonyl,
   j) N(H)-1-Methylcyclobutylcarbonyl,
   k) N(H)-Vinylcarbonyl,
   l) N(H)-Allylcarbonyl,
   m) N(H)-Adamantylcarbonyl,
   n) N(H)-Diphenylmethylcarbonyl,
   o) N(H)-Cinnamylcarbonyl,
   p) N(H)-Formyl,
   q) N(H)-Benzoyl,
   r) N(H)-Trityl,
   s) N(H)-p-Methoxyphenyl-diphenylmethyl,
   t) N(H)-di-(p-Methoxyphenyl)-phenylmethyl,
   u) 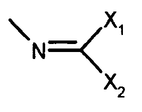 oder
   v) N-(tert-Butoxycarbonyl)₂,
      steht,
L₂ für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl, oder
   d) verzweigtes oder unverzweigtes C₂-C₅ Alkinyl
      steht,
X₁ und X₂ unabhängig voneinander für
   a) verzweigtes oder unverzweigtes C₁-C₅ Alkyl,
   b) substituiertes oder unsubstituiertes Aryl,
   c) substituiertes oder unsubstituiertes Aralkyl, oder
   d) substituiertes oder unsubstituiertes heteroaryl
      steht, und
Z₂ für ein Metallkationenequivalent
   steht,
wobei n = 0, 1, 2 oder 3 ist und
alle Diastereomere und Enantiomere umfasst sind.

Bevorzugt Verbindungen nach Formel VI **dadurch gekennzeichnet, dass** R₈ und R₉ ausgewählt ist aus der Gruppe Wasserstoff, substituiertes oder unsubstituiertes ¹⁸F-C₆-C₁₀ Aryl, substituiertes oder unsubstituiertes ¹⁸F-C₅-C₁₀ Heteroaryl, und substituiertes oder unsubstituiertes ¹⁸F-C₃-C₆ Cyclo-Alkyl, mit der Maßgabe, dass einer der Substituenten R₈ oder R₉ genau ein ¹⁸F Isotop beinhaltet und der jeweils andere Substituent Wasserstoff ist.

### Verbindungen nach Formel VI

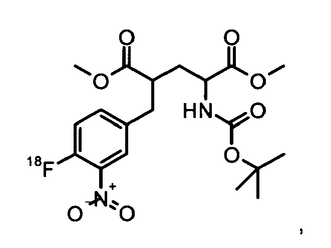

Verbindungen nach Formel VI zur Verwendung als Arzneimittel.
Verbindungen nach Formel VI zur Verwendung für die Bildgebung bei Tumorerkrankungen. Verwendung von Verbindungen nach Formel VI zur Herstellung eines Arzneimittels für die Bildgebung bei Tumorerkrankungen.

Verfahren zur Herstellung von Verbindungen der allgemeinen Formel (VI) indem
- eine Verbindung nach Formel (VII) mit F-18 Fluorid umgesetzt wird.
   In einem weiteren Aspekt bezieht sich die vorliegende Erfindung auf Verbindungen der allgemeinen Formel (VII) 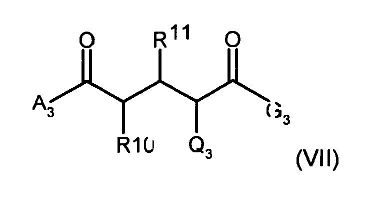
worin
A₃ für
   a) verzweigtes oder unverzweigtes C₁-C₅ Alkoxy,
   b) verzweigtes oder unverzweigtes Hydroxy C₁-C₅ Alkoxy,
   c) verzweigtes oder unverzweigtes O-C₁-C₅ Alkyl-(O-C₁-C₄ alkyl)ₙ-O-C₁-C₄ alkyl
   d) N(C₁-C₅ Alkyl)₂,
   e) NH₂,
   f) N(H)-L₃ oder
   g) O-L₃
      steht,
G₃ für
   a) O-Z₃,
   b) verzweigtes oder unverzweigtes O-C₁-C₅ Alkyl,
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl,
   e) verzweigtes oder unverzweigtes O-C₂-C₅ Alkinyl, oder
   f) Triphenylmethoxy
      steht,
R¹⁰ und R¹¹ für
   a) Wasserstoff,
   b) substituiertes oder unsubstituiertes E₁-C₆-C₁₀ mono- oder bicyclisches Aryl,
   c) substituiertes oder unsubstituiertes E₁-C₅-C₁₀ mono- oder bicyclisches Heteroaryl,
   d) substituiertes oder unsubstituiertes E₁-C₃-C₆ Cyclo-Alkyl,
   e) Hydroxyl,
   f) verzweigtes oder unverzweigtes C₁-C₅ Alkyl oder
   g) verzweigtes oder unverzweigtes C₁-C₅ Alkoxy
      steht, mit der Maßgabe, dass genau einer der Substituenten R¹⁰ oder R¹¹ ein E₁ beinhaltet und der jeweils andere Substituent kein E₁ beinhaltet,
E₁ für
   a) Chloro,
   b) Bromo,
   c) Methansulfonyloxy,
   d) Trifluormethansulfonyloxy,
   e) Nonafluorbutyloxy,
   f) Tosyloxy, oder
   g) lodo
      steht,
Q₂ für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   g) N(H)-2,2,2-Trichlorethoxycarbonyl,
   h) N(H)-1,1-Dimethylpropinyl,
   i) N(H)-1-Methyl-1-phenyl-ethoxycarbonyl,
   j) N(H)-1-Methyl-1-(4-biphenylyl)-ethoxycarbonyl,
   k) N(H)-Cyclobutylcarbonyl,
   l) N(H)-1-Methylcyclobutylcarbonyl,
   m) N(H)-Vinylcarbonyl,
   n) N(H)-Allylcarbonyl,
   o) N(H)-Adamantylcarbonyl,
   p) N(H)-Diphenylmethylcarbonyl,
   q) N(H)-Cinnamylcarbonyl,
   r) N(H)-Formyl,
   s) N(H)-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 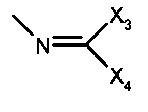 oder
   x) N-(tert-Butoxycarbonyl)₂,
      steht,
L₃ für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkylkO-C₁-C₄ alkyl oder
   d) verzweigtes oder unverzweigtes C₂-C₅ Alkinyl
      steht,
X₃ and X₄ unabhängig voneinander für
   a) verzweigtes oder unverzweigtes C₁-C₅ Alkyl,
   b) substituiertes oder unsubstituiertes Aryl
   c) substituiertes oder unsubstituiertes Alkylaryl oder
   d) substituiertes oder unsubstituiertes Heteroaryl
      steht,
Z₃ für ein Metallkationenequivalent steht,
wobei n = 0, 1, 2 oder 3 ist und
alle Diastereomere und Enantiomere umfasst sind.

Verwendung von Verbindungen der Formel (VII) zur Herstellung von Verbindungen der Formel (V) oder (VI): 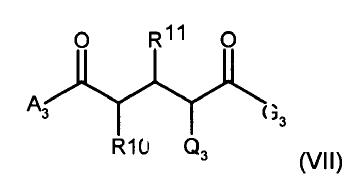 worin
A₃ für
   a) verzweigtes oder unverzweigtes C₁-C₅ Alkoxy,
   b) verzweigtes oder unverzweigtes Hydroxy C₁-C₅ Alkoxy,
   c) verzweigtes oder unverzweigtes O-C₁-C₅ Alkyl-(O-C₁-C₄ alkyl)ₙ-O-C₁-C₄ alkyl
   d) N(C₁-C₅ Alkyl)₂,
   e) NH₂,
   f) N(H)-L₃ oder
   g) O-L₃
      steht,
G₃ für
   a) O-Z₃,
   b) verzweigtes oder unverzweigtes O-C₁-C₅ Alkyl,
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl,
   e) verzweigtes oder unverzweigtes O-C₂-C₅ Alkinyl, oder
   f) Triphenylmethoxy
      steht,
R¹⁰ und R¹¹ für
   a) Wasserstoff,
   b) substituiertes oder unsubstituiertes E₁-C₆-C₁₀ mono- oder bicyclisches Aryl,
   c) substituiertes oder unsubstituiertes E₁-C₅-C₁₀ mono- oder bicyclisches Heteroaryl,
   d) substituiertes oder unsubstituiertes E₁-C₃-C₆ Cyclo-Alkyl,
   e) Hydroxyl,
   f) verzweigtes oder unverzweigtes C₁-C₅ Alkyl oder
   g) verzweigtes oder unverzweigtes C₁-C₅ Alkoxy
      steht, mit der Maßgabe, dass genau einer der Substituenten R¹¹ oder R¹² ein E₁ beinhaltet und der jeweils andere Substituent kein E₁ beinhaltet,
E₁ für
   a) Chloro,
   b) Bromo,
   c) Methansulfonyloxy,
   d) Trifluormethansulfonyloxy,
   e) Nonafluorbutyloxy,
   f) Tosyloxy, oder
   g) lodo
      steht,
Q₂ für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   g) N(H)-2,2,2-Trichlorethoxycarbonyl,
   h) N(H)-1,1-Dimethylpropinyl,
   i) N(H)-1-Methyl-1-phenyl-ethoxycarbonyl,
   j) N(H)-1-Methyl-1-(4-biphenylyl)-ethoxycarbonyl,
   k) N(H)-Cyclobutylcarbonyl,
   l) N(H)-1-Methylcyclobutylcarbonyl,
   m) N(H)-Vinylcarbonyl,
   n) N(H)-Allylcarbonyl,
   o) N(H)-Adamantylcarbonyl,
   p) N(H)-Diphenylmethylcarbonyl,
   q) N(H)-Cinnamylcarbonyl,
   r) N(H)-Formyl,
   s) N(H)-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 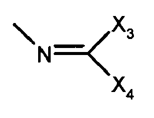 oder
   x) N-(tert-Butoxycarbonyl)₂,
      steht,
L₃ für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl oder
   d) verzweigtes oder unverzweigtes C₂-C₅ Alkinyl
      steht,
X₃ and X₄ unabhängig voneinander für
   a) verzweigtes oder unverzweigtes C₁-C₅ Alkyl,
   b) substituiertes oder unsubstituiertes Aryl,
   c) substituiertes oder unsubstituiertes Alkylaryl oder
   d) substituiertes oder unsubstituiertes Heteroaryl
      steht,
Z₃ für ein Metallkationenequivalent steht,
   wobei n = 0, 1, 2 oder 3 ist und
   alle Diastereomere und Enantiomere umfasst sind.

In einem weiteren Aspekt bezieht sich die vorliegende Erfindung auf Verbindungen der allgemeinen Formel (VIII) 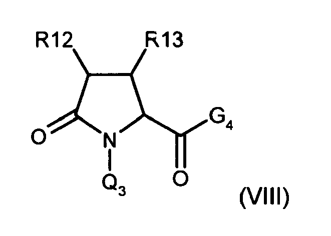 worin
G₄ für
   a) verzweigtes oder unverzweigtes O-C₁-C₅ Alkyl,
   b) verzweigtes oder unverzweigtes O-C₂-C₅ Alkenyl,
   c) verzweigtes oder unverzweigtes O-C₁-C₅ Alkyl-(O-C₁-C₄ alkyl)ₙ-O-C₁-C₄ alkyl,
   d) verzweigtes oder unverzweigtes O-C₂-C₅ Alkinyl, oder
   e) triphenylmethoxy
      steht,
R¹² und R¹³ für
   a) Wasserstoff,
   b) Hydroxyl,
   c) verzweigtes oder unverzweigtes C₁-C₅ Alkyl,
   d) verzweigtes oder unverzweigtes C₁-C₅ Alkoxy oder
   e) R¹⁴-E₂
      steht, mit der Maßgabe, dass genau einer der Substituenten R¹² oder R¹³ ein E₂ beinhaltet und der jeweils andere Substituent kein E₂ beinhaltet,
E₂ für
   a) Chloro,
   b) Bromo,
   c) Methansulfonyloxy,
   d) Trifluormethansulfonyloxy,
   e) Nonafluorbutyloxy,
   f) Tosyloxy oder
   g) lodo
      steht,
R¹⁴ für
   a) substituiertes oder unsubstituiertes C₆-C₁₀ mono- oder bicyclisches Aryl,
   b) substituiertes oder unsubstituiertes C₅-C₁₀ mono- oder bicyclisches Heteroaryl,
   c) substituiertes oder unsubstituiertes C₃-C₆ Cyclo-Alkyl,
      steht,
Q₃ für
   a) N-tert-Butoxycarbonyl,
   b) N-Allyloxycarbonyl,
   c) N-Benzyloxycarbonyl,
   d) N-Ethoxycarbonyl,
   e) N-Methoxycarbonyl,
   f) N-Propoxycarbonyl,
   g) N-2,2,2-Trichlorethoxycarbonyl,
   h) Wasserstoff,
   i) N-1-Methyl-1-phenyl-ethoxycarbonyl,
   j) N-1-Methyl-1-(4-biphenylyl)-ethoxycarbonyl,
   k) N-cyclobutylcarbonyl,
   l) N-1-Methylcyclobutylcarbonyl,
   m) N-Vinylcarbonyl,
   n) N-Allylcarbonyl,
   o) N-Adamantylcarbonyl,
   p) N-Diphenylmethylcarbonyl,
   q) N-Cinnamylcarbonyl,
   r) N-Formyl, oder
   s) N-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 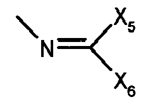 oder
   x) N-(tert-Butoxycarbonyl)₂,
      steht,
X₅ and X₆ unabhängig voneinander für
   a) verzweigtes oder unverzweigtes C₁-C₅ Alkyl,
   b) substituiertes oder unsubstituiertes Aryl
   c) substituiertes oder unsubstituiertes Alkylaryl, oder
   d) substituiertes oder unsubstituiertes Heteroaryl
      steht,
   wobei n = 0, 1, 2 oder 3 ist und
   alle Diastereomere und Enantiomere umfasst sind.

Verwendung von Verbindungen der Formel (VIII) zur Herstellung von Verbindungen der Formel (V) oder (VI): 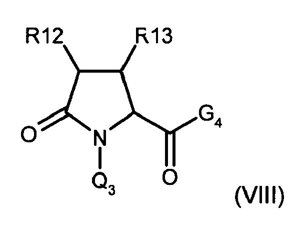 worin
G₄ für
   a) verzweigtes oder unverzweigtes O-C₁-C₅ Alkyl,
   b) verzweigtes oder unverzweigtes O-C₂-C₅ Alkenyl,
   c) verzweigtes oder unverzweigtes O-C₁-C₅ Alkyl-(O-C₁-C₄ alkyl)ₙ-O-C₁-C₄ alkyl,
   d) verzweigtes oder unverzweigtes O-C₂-C₅ Alkinyl, oder
   e) triphenylmethoxy
      steht,
R¹² und R¹³ für
   a) Wasserstoff,
   b) Hydroxyl,
   c) verzweigtes oder unverzweigtes C₁-C₅ Alkyl,
   d) verzweigtes oder unverzweigtes C₁-C₅ Alkoxy oder
   e) R¹⁴-E₂
      steht, mit der Maßgabe, dass genau einer der Substituenten R¹² oder R¹³ ein E₂ beinhaltet und der jeweils andere Substituent kein E₂ beinhaltet,
E₂ für
   a) Chloro,
   b) Bromo,
   c) Methansulfonyloxy,
   d) Trifluormethansulfonyloxy,
   e) Nonafluorbutyloxy,
   f) Tosyloxy oder
   g) lodo
      steht,
R₁₄ für
   a) substituiertes oder unsubstituiertes C₆-C₁₀ mono- oder bicyclisches Aryl,
   b) substituiertes oder unsubstituiertes C₅-C₁₀ mono- oder bicyclisches Heteroaryl, oder
   c) substituiertes oder unsubstituiertes C₃-C₆ Cyclo-Alkyl,
      steht,
Q₃ für
   a) N-tert-Butoxycarbonyl,
   b) N-Allyloxycarbonyl,
   c) N-Benzyloxycarbonyl,
   d) N-Ethoxycarbonyl,
   e) N-Methoxycarbonyl,
   f) N-Propoxycarbonyl,
   g) N-2,2,2-Trichlorethoxycarbonyl,
   h) Wasserstoff,
   i) N-1-Methyl-1-phenyl-ethoxycarbonyl,
   j) N-1-Methyl-1-(4-biphenylyl)-ethoxycarbonyl,
   k) N-cyclobutylcarbonyl,
   l) N-1-Methylcyclobutylcarbonyl,
   m) N-Vinylcarbonyl,
   n) N-Allylcarbonyl,
   o) N-Adamantylcarbonyl,
   p) N-Diphenylmethylcarbonyl,
   q) N-Cinnamylcarbonyl,
   r) N-Formyl, oder
   s) N-Benzoyl,
   t) N(H)-Trityl,
   u) N(H)-p-Methoxyphenyl-diphenylmethyl,
   v) N(H)-di-(p-Methoxyphenyl)-phenylmethyl,
   w) 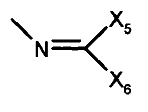 oder
   x) N-(tert-Butoxycarbonyl)₂,
      steht,
X₅ and X₆ unabhängig voneinander für
   a) verzweigtes oder unverzweigtes C₁-C₅ Alkyl,
   b) substituiertes oder unsubstituiertes Aryl
   c) substituiertes oder unsubstituiertes Alkylaryl, oder
   d) substituiertes oder unsubstituiertes Heteroaryl
      steht,
   wobei n = 0, 1, 2 oder 3 ist und
   alle Diastereomere und Enantiomere umfasst sind.

Die Erfindung bezieht sich auf Bildgebung-Kit enthaltend Verbindungen der allgemeinen Formel VII oder VIII.

Die Erfindung bezieht sich auf Pharmazeutische Zusammensetzung enthaltend Verbindungen der allgemeinen Formel V, VI, VII oder VIII und geeignete pharmazeutische Träger Substanzen.

Die Erfindung bezieht sich auf Verbindungen nach Formel I, II, V oder VI **dadurch gekennzeichnet dass** die Verbindungen für Bildgebung in einem Dosisbereich von 37 - 600 MBq geeignet sind.

Besonders bevorzugte Verbindungen **dadurch gekennzeichnet dass** die Verbindungen in einem Dosisbereich von 150 MBq - 370 MBq besonders geeignet sind.

Bevorzugt für die Einführung des ¹⁸F Isotops sind
4,7,13,16,21,24-Hexaoxa-1,10-diazabicyclo[8.8.8]-hexacosane K18F (Kronenethersalz Kryptofix K18F),
K¹⁸F,
H¹⁸F,
KH¹⁸F₂,
Cs¹⁸F,
Na¹⁸F oder
¹⁸F tetraalkylammonium salz (z.b [F-18] tetrabutylammonium fluoride).

Wenn ein oder mehrere chirale Zentren in einer Verbindung des vorliegenden Erfindungsgegenstandes der Formel (I), bis Formel (VIII), vorhanden ist bzw. sind, so sollen alle Formen dieses Isomers, einschließlich beider Enantiomere und aller möglichen Diastereomere, hierin beinhaltet sein. Verbindungen, die mindestens ein chirales Zentrum enthalten, können als racemische Mischung, gegebenenfalls als Diastereomeren- oder Diastereomeren-angereicherte Mischung oder als Enantiomeren-angereicherte Mischung eingesetzt werden. Die racemische, enantiomeren-angereicherte Mischung oder Diastereomerenmischung kann gegebenenfalls nach den dem Fachmann bekannten Methoden getrennt werden, so dass die Enantiomere oder Diastereomere einzeln eingesetzt werden können. In solchen Fällen, in denen eine Kohlenstoff-Kohlenstoff-Doppelbindung vorliegt, so sind beide Isomere "cis" und "trans" Teil der vorliegenden Erfindung. In solchen Fällen, in denen tautomere Formen vorliegen können, wie z. Bsp. Keto-enol Tautomerie, sind alle tautomeren Formen in der vorliegenden Erfindung beinhaltet, wobei diese Formen im Gleichgewicht oder bevorzugt in einer Form vorliegen können.

Die Verbindungen der allgemeinen Formel I bis VIII und ihre bevorzugten Ausführungsformen werden verwendet als Arzneimittel.

Die erfindungsgemäßen Verbindungen der allgemeinen Formel I, II, V oder VI und ihre bevorzugten Ausführungsformen werden verwendet in der Diagnose von physiologischen oder pathologischen Zuständen.

Bevorzugt finden diese Verbindungen Anwendung in der nicht-invasiven PET-basierten Diagnose am menschlichen oder tierischen Körper.

Besonders bevorzugt finden die erfindungsgemäßen Verbindungen der allgemeinen Formel I, II, V oder VI und ihre bevorzugten Ausführungsformen Anwendung in der Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich hormonabhängigem und hormonunabhängigem Brustkrebs, Plattenepithelkarzinom, neurologische Krebserkrankungen einschließlich Neuroblastom, Gliom, Astrocytom, Osteosarcom, Meningiom; Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

Die erfindungsgemäßen Verbindungen der allgemeinen Formel I, II, V oder VI und ihre bevorzugten Ausführungsformen werden verwendet zur Herstellung eines Arzneimittels für die Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nichtkleinzelliges Bronchialkarzinom, dysplastisches orales Mucosa-Karzinom, invasiver Oral-Krebs; Brustkrebs, einschließlich Hormon-abhängigem und Hormon-unabhängigem Brustkrebs, Plattenepithelkarzinom, neurologische Krebserkrankungen einschließlich Neuroblastom, Gliom, Astrocytom, Osteosarcom, Meningiom, Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

Die Erfindung betrifft pharmazeutische Präparate, welche mindestens eine Verbindung der Formel I oder II sowie einen pharmazeutisch verträglichen Träger enthalten.

Zur Verwendung der Verbindungen der Formel I oder II als Arzneimittel werden diese in die Form eines pharmazeutischen Präparats gebracht, welches neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält.

Die Erfindung betrifft eine Einrichtung (Kit) enthaltend mindestens eine Verbindung der Formel I, bis VIII.

Der Begriff "Aryl", so wie er hier selbst stehend oder als Teil einer anderen Gruppe angewendet wird, bezieht sich auf mono-zyklische oder zweifach-zyklische aromatische Gruppen, die sechs bis zwölf Kohlenstoffatome im Ring beinhalten können, wie z. Bsp. Phenyl oder Naphtyl, und in Position 2 beliebig substituiert sein können.

Die Arylgruppen können an jeder geeigneten Stelle, die zu einer stabilen Verbindung führt, substituiert sein durch einen oder mehrere Reste aus der Gruppe: Hydroxy, Halogen, C₁-C₅-Alkyl, C₁-C₅-Alkoxy, Cyano, CF₃, Nitro.

Als Substituenten seien Methoxy-, Ethoxy-, Propoxy-,iso-Propoxy, Hydroxy, Fluor-, Chlor-, Brom-, Methyl-, Ethyl-, Propyl-, iso-Propyl oder Trifluormethylgruppen genannt.

Unter Halogen ist jeweils Fluor, Chlor, Brom oder lod zu verstehen.

Der Begriff "Alkyl", so wie er hier selbst stehend oder als Teil einer anderen Gruppe angewendet wird, bezieht sich auf C₁-C₆-Alkylgruppen und können geradkettig oder verzweigt sein und für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe stehen. Eine Methyl- oder Ethylgruppe ist bevorzugt. Unter Alkyl ist jeweils ein geradkettiger oder verzweigter Alkylrest, wie beispielsweise Methyl, Ethyl, Propyl, Isopropyl, Butyl, Isobutyl, sek-Butyl, tert-Butyl, Pentyl, Isopentyl oder Hexyl, Heptyl, Octyl, Nonyl, Decyl, Undecyl, Dodecyl zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, , But-3-en-1-yl, Allyl.

Die Alkinylgruppen können geradkettig oder verzweigt sejn und beispielsweise Ethinyl, - CH₂-C≡CH, -CH₂-C≡CH, -C≡C-CH₃, -CH(CH₃)-C≡CH, -C≡C-CH₂(CH₃), -C(CH₃)₂-C≡CH, -C≡C-CH(CH₃)₂-, -CH(CH₃)-C≡C-CH₃, , -CH₂-C≡C- CH₂(CH₃) bedeuten.

Die C₁-C₅-Alkoxygruppen können geradkettig oder verzweigt sein und für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. Eine Methoxy- oder Ethoxygruppe ist bevorzugt.

Der Heteroarylrest umfaßt jeweils 5 - 16 Ringatome und kann anstelle eines Kohlenstoffatoms ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.
Beispielsweise seien genannt:
Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc.
   und
Benzoderivate davon, wie z. B.
   Benzofuranyl, Benzothienyl, Benzothiazol, Benzoxazolyl, Benzimidazolyl, Indazolyl, Indolyl, Isoindolyl, etc.;
   oder
Pyridyl, Pyridazinyl, Pyrimidinyl, Pyrazinyl, Triazinyl, etc. und Benzoderivate davon, wie z. B.
Chinolyl, Isochinolyl, etc.;
   oder
   Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl.

Erfindungsgemäße Verbindungen, bei denen das [F-18]-Isotop über eine Alkoxygruppe in 4-Position des Glutaminsäuregerüstes positioniert ist, wie z. B. bei 4-[F-18]Fluorohexoxyglutaminsäure (**1**), lassen sich wie in Schema 9 dargestellt, herstellen. So gelingt beispielshalber die saure Abspaltung der Schutzgruppen der Verbindung **2** oder (**3**) um die erfindungsgemäße Verbindung 4-[F-18]Fluorohexoxy-glutaminsäure (**1**) zu erhalten.

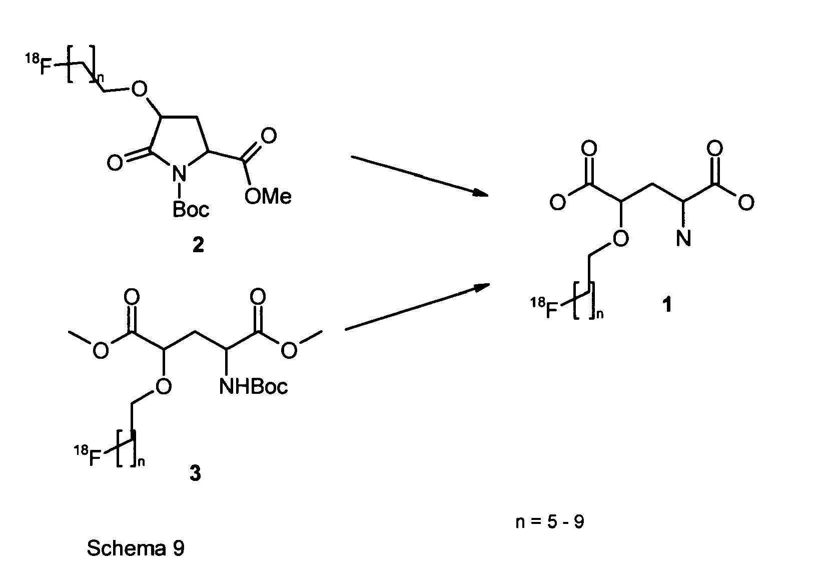

Dabei können verschiedene organische (z. B. Trifluoressigsäure), vor allem aber anorganische Säuren wie z. B. Bromwasserstoffsäure, Salzsäure, Schwefelsäure, Perchlorsäure oder Phosphorsäure zum Einsatz kommen. Weiterhin ist auch eine basische Ringöffnung von **2** möglich mit Lithiumhydroxid, Natriumhydroxid, Kaliumhydroxid etc. (S. Baker et al. Tetrahedron Lett. 1998, 39, 2815-2818.).
Die Reinigung der erfindungsgemäßen Verbindung **1** nach Formel (I) ist per HPLC möglich, wobei verschiedene Reinigungsschritte prinzipiell vorgeschaltet und nachgeschaltet sein können, wie z. B. die Reinigung über eine RP-C18 Kartusche oder andere Trennmaterialien.

Die radiochemische Fluorierung von Tosylat **4** dessen Synthese analog der in der Literatur beschriebenen Methode (N. Sharma et al. Tetrahedron Lett. 2004, 45, 1403-1406.) aus **5** erfolgte, zum [F-18]-markierten Glutaminsäurederivat **2** ist nach den dem Fachmann bekannten Methoden durchführbar (s. Schema 10).

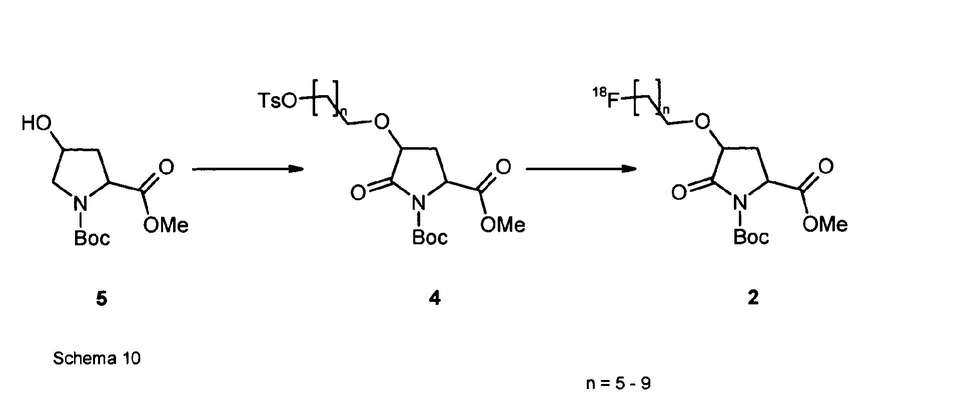

Dabei kann Verbindung **2** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **2** muss üblicherweise keiner Reinigung unterzogen werden, sondern kann umgehend mit den für die Umsetzung von **2** nach **1** beschriebenen Methoden behandelt werden. Eine Reinigung der Verbindung **2** ist aber prinzipiell möglich, bevorzugt mittels einer präparativen HPLC mit einer unpolaren Phase, wie z. B. RP C-18. Außerdem ist eine Reinigung mittels Kartuschen möglich.

Die radiochemische Fluorierung von Tosylat **6** dessen Synthese analog der in der Literatur beschriebenen Methode (X. Zhang Tetrahedron Lett. 2001, 42, 5335-5338.) aus **4** erfolgte, zum [F-18]-markierten Glutaminsäurederivat **3** ist nach den dem Fachmann bekannten Methoden durchführbar (s. Schema 11).

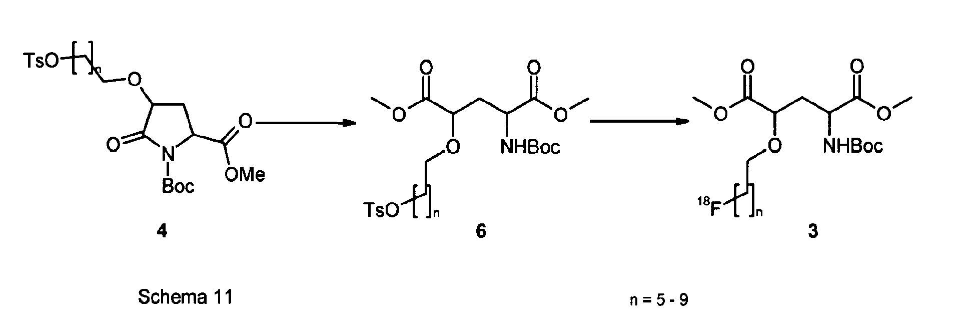

Dabei kann Verbindung **6** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **3** muss üblicherweise keiner Reinigung unterzogen werden, sondern kann umgehend mit den für die Umsetzung von **3** nach **1** beschriebenen Methoden behandelt werden. Eine Reinigung der Verbindung **3** ist aber prinzipiell möglich, bevorzugt mittels einer präparativen HPLC mit einer unpolaren Phase, wie z. B. RP C-18. Außerdem ist eine Reinigung mittels Kartuschen möglich.

Die Synthese von F-19 Referenzverbindungen **7, 8** und **9** kann wie in Schema 12 dargestellt erfolgen.

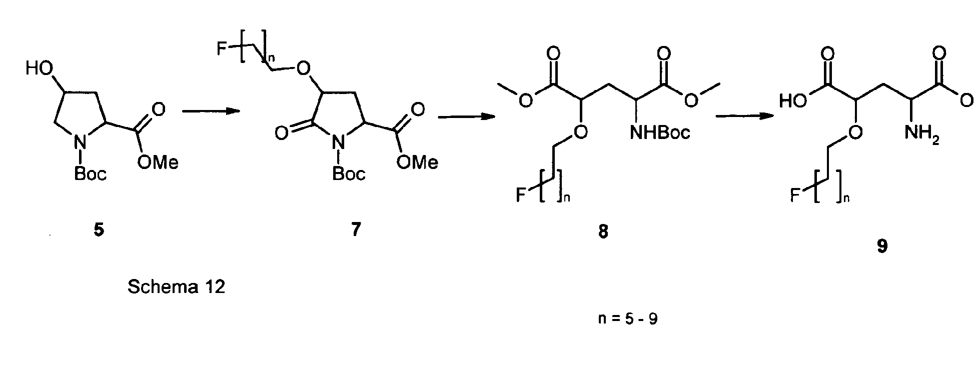

**7** kann durch Alkylierung und Oxidation des Hydroxyprolinderivats **5** erhalten werden. Vorteilhaft erweist sich bei der Darstellung von F-19 Referenzverbindungen auch die Darstellung der Fluoride aus den analogen Hydroxyverbindungen mittels DAST (Diethylaminoschwefeltrifluorid) nach den dem Fachmann bekannten Verfahren wie z.B. in Beispiel **2c** beschrieben.

Durch Ringöffnung des Pyroglutaminderivats **7** wird die offenkettige Referenzverbindung **8** erhalten. Die saure Abspaltung der Schutzgruppen führt zum Glutaminsäurederivat **9.**

Erfindungsgemäße Verbindungen, bei denen das [F-18]-Isotop über eine Alkylgruppe in 4-Position des Glutaminsäuregerüstes positioniert ist, wie z. B. bei 4-[F-18]Fluorohexylglutaminsäure (**10**) oder 4-[F-18]Fluorooctyl-glutaminsäure (**11**) lassen sich wie in Schema 13 dargestellt, herstellen. So gelingt beispielshalber die saure Abspaltung der Schutzgruppen der Verbindungen **12** und **13,** um die erfindungsgemäßen Verbindung 4-[F-18]Fluorohexyl-glutaminsäure (**10**) oder 4-[F-18]Fluorooctyl-glutaminsäure (**11**) zu erhalten.

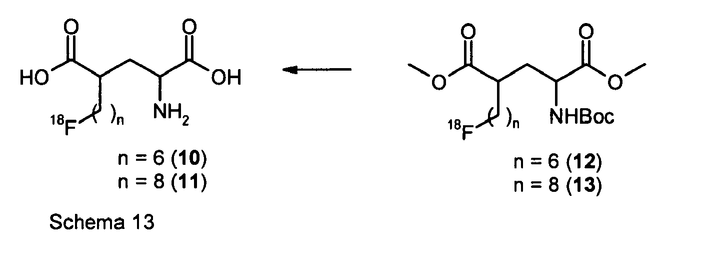

Dabei können verschiedene organische (z. B. Trifluoressigsäure), vor allem aber anorganische Säuren wie z. B. Bromwasserstoffsäure, Salzsäure, Schwefelsäure, Perchlorsäure oder Phosphorsäure zum Einsatz kommen. Die Reinigung der erfindungsgemäßen Verbindung **10** und **11** nach Formel (I) ist per HPLC möglich, wobei verschiedene Reinigungsschritte prinzipiell vorgeschaltet und nachgeschaltet sein können, wie z. B. die Reinigung über eine RP-C18 Kartusche oder andere Trennmaterialien.

Die radiochemische Fluorierung von Bromid **14** oder Tosylat **15,** deren Synthese analog der in der Literatur beschriebenen Methode (S. Hanessian, et al. J. Org. Chem. 2005, 70, 5070-5085) aus **16** erfolgte, zu den [F-18]-markierten Glutaminsäurederivaten **12** und **13** ist nach den dem Fachmann bekannten Methoden durchführbar (s. Schema 14).

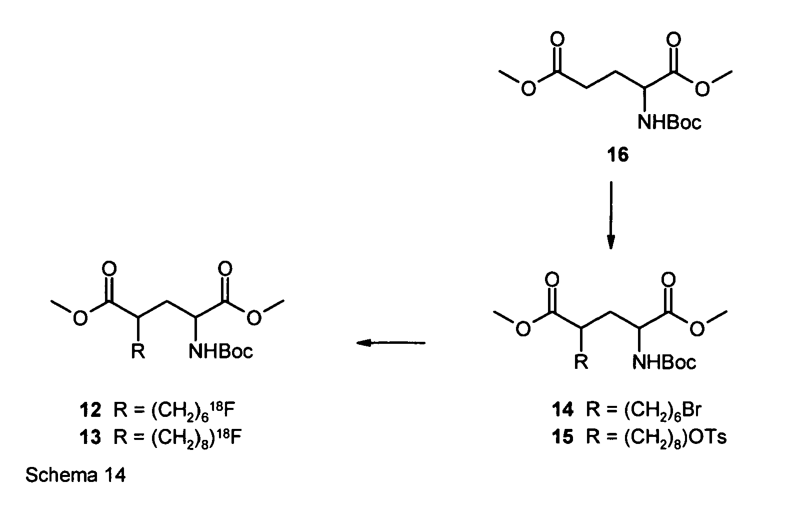

Dabei können die Verbindungen **14** und **15** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Die Verbindungen **12** und **13** müssen üblicherweise keiner Reinigung unterzogen werden, sondern können umgehend mit den für die Umsetzung von **12** nach **10** bzw. **13** nach **11** beschriebenen Methoden behandelt werden. Eine Reinigung der Verbindungen **12** bzw. 13 ist aber prinzipiell möglich, bevorzugt mittels einer präparativen HPLC mit einer unpolaren Phase, wie z. B. RP C-18.

Die Synthese der F-19 Referenzverbindungen **17** und **18** kann durch Alkylierung des Glutaminsäurederivats **16** erfolgen (Schema 15).

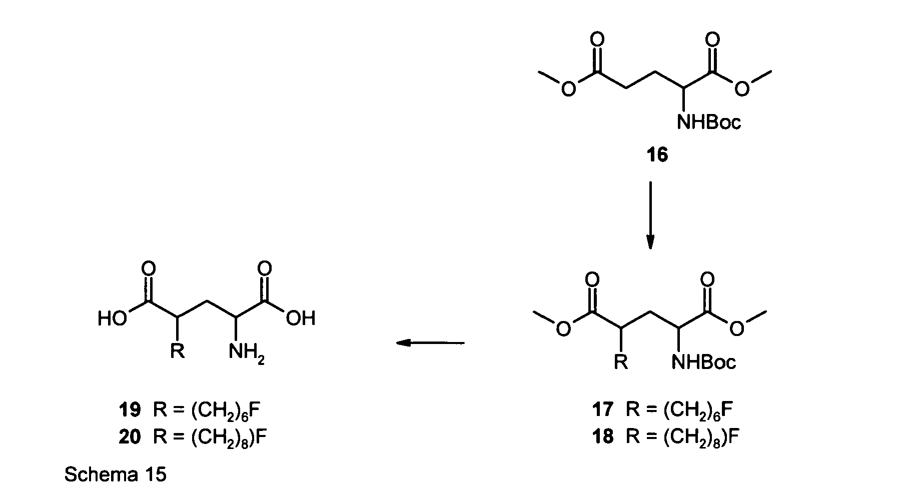

Die Verbindung **16** kann auch mit lodiden, vorzugsweise mit Diiodiden analog Beispiel **1a** alkyliert werden. Man erhält dann in einem Schritt aus kommerziell verfügbarem Glutaminsäurederivat **16** einen zur radiochemischen Fluorierung geeigneten Präkursor.

Die Abspaltung der Schutzgruppen führt zu den Fluoralkylierten Glutaminsäurederivaten **19** und **20.**

Als erfindungsgemäße Präkursoren eignen sich auch aromatische Nitroverbindungen wie in Beispiel 3 ausgeführt.
Die Einführung von Cycloalkyl-substituenten erfolgt nach den dem Fachmann bekannten Verfahren, z.B. durch Alkylierung des Glutaminsäurederivats **16** wie in Beispiel **2a** beschrieben.

### Beispiele:

### Beispiel 1

### 2-Amino-4-(6-fluorohexyl)-pentandisäure

a) (2S,4S)-2-tert-Butoxycarbonylamino-4-(6-iodohexyl)-pentandisäure-dimethylester (Präkursor für (2S,4S)-2-Amino-4-(6-fluorohexyl)-pentandisäure) 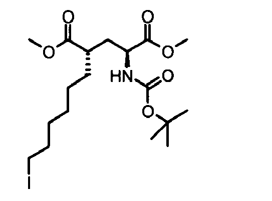 5,51 g (20 mmol) Boc-L-Glutaminsäure dimethylester (Advanced Chemtech) werden in 60 mL Tetrahydrofuran (THF) gelöst und auf -70°C gekühlt. Innerhalb einer Stunde werden bei dieser Temperatur 44 mL (44 mmol) 1 M Lösung von Lithium-bis(trimethylsilyl)amid in THF zugetropft und 2 Stunden bei -70°C nachgerührt. Anschließend werden 20,28 g (60 mmol) 1,6-Diiodhexan zugetropft und nach 2 h bei dieser Temperatur wird das Kältebad entfernt und 100 mL 2 N Salzsäure und 300 mL Essigester zugegeben. Die organische Phase wird abgetrennt, mit Wasser neutral gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 0,2 g (2,1 %)
Elementaranalyse:

| | | | | |
|---|---|---|---|---|
| ber.: | C 44.54 | H 6.65 | I 26.15 | N 2.89 |
| gef: | C 44.28 | H 6.75 | I 25.83 | N 3.04 |

b) (2S,4S)-2-tert-Butoxycarbonylamino-4-(6-fluorohexyl)-pentandisäure-dimethylester 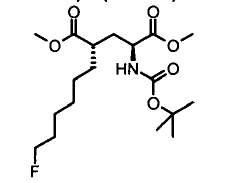 Zu 0.49 g (1 mmol) der in Beispiel **1a** beschriebenen Verbindung in 30 mL Acetonitril wird eine Lösung von 152 mg (1,12 mmol) Silberfluorid in 1,5 mL Wasser zugegeben und über Nacht bei 40°C gerührt. Die erhaltene Suspension wird filtriert, die Lösung zur Trockne eingedampft und das so erhaltene Rohprodukt wird mit einem Hexan/Essigester-Gradienten an Kieselgel chromatographiert und die entsprechenden Fraktionen vereinigt und eingeengt.
Ausbeute: 132 mg (35 %)
Elementaranalyse:

| | | | | |
|---|---|---|---|---|
| ber.: | C 57.28 | H 8.55 | F 5.03 | N 3.71 |
| gef: | C 57.03 | H 8.41 | F 4.80 | N 3.82 |

c) (2S,4S)-2-Amino-4-(6-fluorohexyl)-pentandisäure 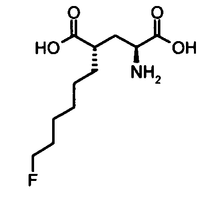 26,4 mg (0,07 mmol) der in Beispiel **1b** beschriebenen Verbindung werden in 2 mL THF gelöst, mit 1 mL 1 N Natronlauge versetzt und 4 h bei Raumtemperatur gerührt. Anschließend wird zur Trockne eingeengt und das erhaltene Rohprodukt in ca. 20 mL 3 N Chlorwasserstoff in Diethylether gelöst, über Nacht gerührt, eingeengt und mehrfach mit Diethylether nachdestilliert. Das so erhaltene Rohprodukt wird mit einem Wasser/Methanol-Gradienten an C18-Kieselgel chromatographiert und die entsprechenden Fraktionen vereinigt und eingeengt.
Ausbeute: 5,8 mg (33 %)
Elementaranalyse (berechnet auf die wasserfreie Verbindung):

| | | | | |
|---|---|---|---|---|
| ber.: | C 53.00 | H 8.09 | F 7.62 | N 5.62 |
| gef: | C 52.68 | H 8.33 | F 7.24 | N 5.41 |

d) (2S,4S)-2-tert-Butoxycarbonylamino-4-(6-[F-18]fluorohexyl)-pentandisäuredimethylester 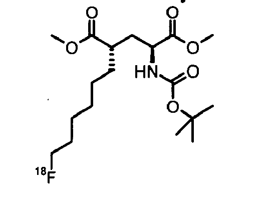 [F-18]Fluorid wurde über die [O-18](p,n)[F-18]-Reaktion in einem Zyklotron hergestellt. Die Isotopenlösung (5,3 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120°C in einem Stickstoffstrom unter Zugabe von Acetonitril (dreimal 1 mL) entfernt.
5 mg (10.0 µmol) 2-tert-Butoxycarbonylamino-4-[6-iodo-hexyl]-pentandisäure-dimethylester (1a) in 1 mL Acetonitril wurden zugegeben und die resultierende Mischung wurde 10 min bei 110°C gerührt. Nach Abkühlen auf ca. 60 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 80 min wurden 1,3 GBq (41 % d.c.) (2S,4S)-2-tert-Butoxycarbonylamino-4-(6-[F-18]fluorohexyl)-pentandisäuredimethylester erhalten (1d).
e) (2S,4S)-2-Amino-4-(6-[F-18]fluorohexyl)-pentandisäure 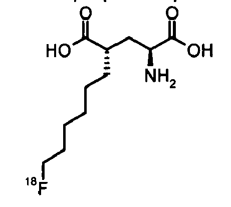 1,2 GBq (2S,4S)-2-tert-Butoxycarbonylamino-4-(6-[F-18]fluorohexyl)-pentandisäuredimethylester (1d) in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 700 µL 2N NaOH neutralisiert.
Es wurden 1,0 GBq (83 % d.c.) (2S,4S)-2-Amino-4-(6-[F-18]fluorohexyl)-pentandisäure (1e) erhalten.

### Beispiel 2

### 2-Amino-4-(3-fluor-cyclobutoxy)-pentandisäure

a) 4-(3-Benzyloxy-cyclobutoxy)-5-oxo-pyrrolidine-1,2-disäure 1-tert-butyl ester 2-methyl ester 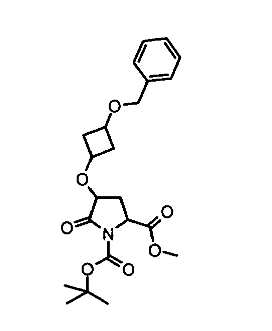 0,98 g (4 mmol) (2S,4S)-4-Hydroxypyrrolidin-1,2-disäure 1-tert-butyl ester 2-methyl ester (ALDRICH) werden in 10 mL Dichlormethan gelöst und im Eisbad gekühlt. Nach Zugabe von 1,03 g Methansulfonsäure 3-benzyloxy-cyclobutyl ester (WO 9937644 v. 21.1.1998) und 1,36 g (4 mmol) Tetrabutylammonium-hydrogensulfat werden 18 mL 50%ige wässrige Natronlauge zugegeben, 2 Stunden im Eis und über Nacht bei Raumtemperatur gerührt. Nach Zugabe von 200 mL Wasser und 200 mL Dichlormethan wird die organische Phase noch einmal mit Wasser gewaschen, über Natriumsulfat getrocknet, filtriert und das Filtrat eingeengt. Das so erhaltene Rohprodukt wird mit einem Dichlormethan /Essigester-Gradienten an Kieselgel chromatographiert und die entsprechenden Fraktionen vereinigt und eingeengt.
Ausbeute: 67 mg (4,0 %)
Elementaranalyse:

| | | | |
|---|---|---|---|
| ber.: | C 62.99 | H 6.97 | N 3.98 |
| gef: | C 62.70 | H 7.21 | N 4.19 |

b) 5-Oxo-4-[3-(toluol-4-sulfonyloxy)-cyclobutoxy]-pyrrolidin-1,2-disäure 1-tert-butyl ester 2-methyl ester
(Präkursor für 2-Amino-4-(3-fluor-cyclobutoxy)-pentandisäuresäure) 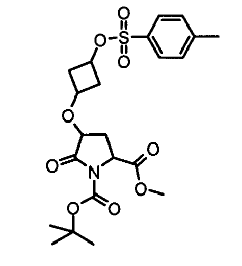 210 mg (0,5 mmol) der in Beispiel **2a** beschriebenen Verbindung werden in 5 mL Methanol gelöst und an Palladium-Katalysator auf Aktivkohle (5%) unter Wasserstoffatmosphäre hydriert. Nach 20 h wird vom Katalysator abgesaugt und die Lösung eingedampft. Der Rückstand wird in Dichlormethan gelöst und im Eisbad gekühlt. Nach Zugabe von 0,30 g (3 mmol) Triethylamin und 115 mg (1 mmol) Methansulfonsäurechlorid wird 2 h im Eis gerührt, eingeengt und das so erhaltene Rohprodukt wird mit einem Hexan/Essigester-Gradienten an Kieselgel chromatographiert und die entsprechenden Fraktionen vereinigt und eingeengt.
Ausbeute: 104 mg (43 %)
Elementaranalyse:

| | | | | |
|---|---|---|---|---|
| ber.: | C 54.65 | H 6.05 | N 2.90 | S 6.63 |
| gef: | C 54.48 | H 6.19 | N 3.01 | S 6.18 |

c) 2-Amino-4-(3-fluoro-cyclobutoxy)-pentandisäure 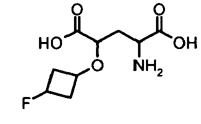 210 mg (0,5 mmol) der in Beispiel **2a** beschriebenen Verbindung werden in 5 mL Methanol gelöst und an Palladium-Katalysator auf Aktivkohle (5%) unter Wasserstoffatmosphäre hydriert. Nach 20 h wird vom Katalysator abgesaugt und die Lösung eingedampft. Die erhaltene Hydroxyverbindung wird ohne weitere Charakterisierung in 10 mL Dichlormethan gelöst und im Eis gekühlt. Nach Zugabe von 0,16 g (1 mmol) Diethylaminoschwefeltrifluorid (DAST) wird 1 h im Eis gerührt, mit Wasser gewaschen und die organische Phase über Natriumsulfat getrocknet, filtriert und das Filtrat eingeengt. Das so erhaltene Rohprodukt des geschützten Fluorids wird mit einem Dichlormethan/Essigester-Gradienten an Kieselgel chromatographiert und die entsprechenden Fraktionen vereinigt und eingeengt.
Der Rückstand wird in 2 mL THF gelöst, mit 1 mL 1 N Natronlauge versetzt und 4 h bei Raumtemperatur gerührt. Anschließend wird erneut zur Trockne eingeengt und die erhaltene Disäure in ca. 20 mL 3 N Chlorwasserstoff in Diethylether gelöst, über Nacht gerührt, eingeengt und mehrfach mit Diethylether nachdestilliert. Das so erhaltene Rohprodukt wird mit einem Wasser/Methanol-Gradienten an C18-Kieselgel chromatographiert und die entsprechenden Fraktionen vereinigt und eingeengt.
Ausbeute: 4,7 mg (4 %)
Elementaranalyse (berechnet auf die wasserfreie Verbindung):

| | | | | |
|---|---|---|---|---|
| ber.: | C 45.96 | H 6.00 | F 8.08 | N 5.95 |
| gef: | C 45.62 | H 6.29 | F 7.87 | N 6.05 |

d) 5-Oxo-4-[3-[F-18]fluoro-cyclobut-1-oxy]-pyrrolidin-1,2-disäure 1-tert-butyl ester 2-methyl ester 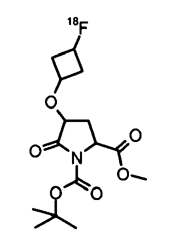 [F-18]Fluorid wurde über die [O-18](p,n)[F-18]-Reaktion in einem Zyklotron hergestellt. Die Isotopenlösung (3.27 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120°C in einem Stickstoffstrom unter Zugabe von Acetonitril (dreimal 1 mL) entfernt.
5 mg (14.9 µmol) 4-[3-(Toluol-4-sulfonyloxy)-cylcobut-1-oxy]-5-oxo-pyrrolidin-1,2-disäure-1-tert-butylester-2-methylester **2b** in 1 mL Dimethylformamid wurden zugegeben und die resultierende Mischung wurde 10 min bei 110 °C gerührt. Nach Abkühlen auf ca. 60 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 75 min wurden 620 MBq (30 % d.c.) 5-Oxo-4-[3-[F-18]fluoro-cyclobut-1-oxy]-pyrrolidin-1,2-disäure 1-tert-butyl ester 2-methyl ester **2d** erhalten.
e) 2-Amino-4-(3-[F-18]fluoro-cyclobut-1-oxy)-pentandisäure 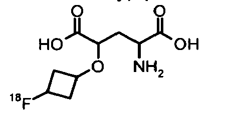 620 MBq 5-Oxo-4-[3-[F-18]fluoro-cyclobut-1-oxy]-pyrrolidin-1,2-disäure 1-tert-butyl ester 2-methyl ester **2d** enthalten in 0.5 mL Acetonitril wurden mit 0.5 mL 6N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 600 µL 4N NaOH neutralisiert. Es wurden 172 MBq (91 % d.c.) 2-Amino-4-(3-[F-18]fluoro-cyclobut-1-oxy)-pentandisäure **2e** erhalten.

### Beispiel 3

### 2-Amino-4-(4-fluoro-3-nitrobenzyl)-pentandisäure

a) 2-tert-Butoxycarbonylamino-4-(3,4-dinitrobenzyl)-pentandisäure dimethyl ester 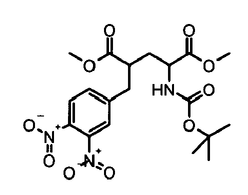 11,01 g (40 mmol) Boc-Glutaminsäure dimethylester (Advanced Chemtech) werden in 160 mL Tetrahydrofuran (THF) gelöst und auf -70°C gekühlt. Innerhalb einer Stunde werden 88 mL (88 mmol) einer 1 M Lösung von Lithium-bis(trimethylsilyl)amid in THF bei dieser Temperatur zugetropft und 2 Stunden bei -70°C nachgerührt. Anschließend werden 20,88 g (80 mmol) 4-Bromomethyl-1,2-dinitrobenzol in 250 mL THF zugetropft und nach 2 h bei dieser Temperatur wird das Kältebad entfernt und 200 mL 2 N Salzsäure und 400 mL Essigester zugegeben. Die organische Phase wird abgetrennt, mit Wasser neutral gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 3,8 g (21 %)
Elementaranalyse:

| | | | |
|---|---|---|---|
| ber.: | C 50.11 | H 5.53 | N 9.23 |
| gef: | C 49.94 | H 5.66 | N 9.40 |

b) 2-Amino-4-(4-fluoro-3-nitrobenzyl)-pentandisäure 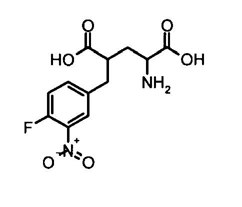 Zu einer Lösung von 455 mg (1 mmol) der in Beispiel **3a** beschriebenen Verbindung in 10 mL Dimethylformamid werden 2,1 mL (2 mmol) einer 1 M Lösung von Tetrabutylammoniumfluorid in THF (Aldrich) zugegeben. Nach 2 h bei Raumtemperatur wird zur Trockne eingedampft, 5 mL und 5 mL Essigester zugegeben, die organische Phase wird abgetrennt, mit Wasser neutral gewaschen, über Natriumsulfat getrocknet, filtriert und das Filtrat eingeengt. Das so erhaltene Rohprodukt der geschützten 4-Fluoro-3-Nitro-verbindung wird mit einem Hexan/Essigester-Gradienten an Kieselgel chromatographiert und die entsprechenden Fraktionen vereinigt und eingeengt.
Der Rückstand wird in 5 mL THF gelöst, mit 2 mL 1 N Natronlauge versetzt und 4 h bei Raumtemperatur gerührt. Anschließend wird erneut zur Trockne eingeengt und die erhaltene Disäure in ca. 30 mL 3 N Chlorwasserstoff in Diethylether gelöst, über Nacht gerührt, eingeengt und mehrfach mit Diethylether nachdestilliert. Das so erhaltene Rohprodukt wird mit einem Wasser/Methanol-Gradienten an C18-Kieselgel chromatographiert und die entsprechenden Fraktionen vereinigt und eingeengt.
Ausbeute: 15 mg (5 %)
Elementaranalyse (berechnet auf die wasserfreie Verbindung):

| | | | | |
|---|---|---|---|---|
| ber.: | C 48.01 | H 4.36 | F 6.33 | N 9.33 |
| gef: | C 47.76 | H 4.44 | F 6.02 | N 9.27 |

c) 2-tert-Butoxycarbonylamino-4-(4-[F-18]fluoro-3-nitrobenzyl)-pentandisäure dimethyl ester 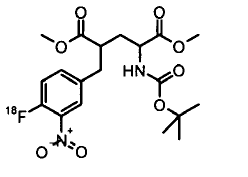 [F-18]Fluorid wurde über die [O-18](p,n)[F-18]-Reaktion in einem Zyklotron hergestellt. Die Isotopenlösung (5.72 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120°C in einem Stickstoffstrom unter Zugabe von Acetonitril (dreimal 1 mL) entfernt.
5 mg (10.9 µmol) 2-tert-Butoxycarbonylamino-4-(3,4-dinitrobenzyl)-pentandisäure dimethyl ester **3a** in 1 mL Acetonitril wurden zugegeben und die resultierende Mischung wurde 15 min bei 130 °C gerührt. Nach Abkühlen auf ca. 60 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 80 min wurden 1,2 GBq (35 % d.c.) 2-tert-Butoxycarbonylamino-4-(4-[F-18]fluoro-3-nitrobenzyl)-pentandisäure dimethyl ester **3c** erhalten.
d) 2-Amino-4-(4-[F-18]fluoro-3-nitrobenzyl)-pentandisäure 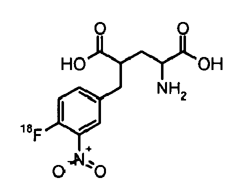 1,1 GBq 2-tert-Butoxycarbonylamino-4-(4-[F-18]fluoro-3-nitrobenzyl)-pentandisäure dimethyl ester **3c** enthalten in 0.5 mL Acetonitril wurden mit 0.5 mL 6N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 600 µL 4N NaOH neutralisiert. Es wurden 945 MBq (86 % d.c.) 2-Amino-4-(4-[F-18]fluoro-3-nitrobenzyl)-pentandisäure 3d erhalten.

### 4 Beispiel 4

a) 4-(6-Iodohexyloxy)-5-oxo-pyrrolidin-1,2-disäure 1-tert-butyl ester 2-methyl ester (Präkursor für 2-Amino-4-(6-fluorohexyloxy)-pentandisäure) 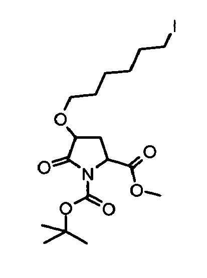 0,98 g (4 mmol) (2S,4S)-4-Hydroxypyrrolidin-1,2-disäure 1-tert-butyl ester 2-methyl ester (ALDRICH) werden in 10 mL Dichlormethan gelöst und im Eisbad gekühlt. Nach Zugabe von 2,03 g (6 mmol) 1,6-Diiodhexan und 1,36 g (4 mmol) Tetrabutylammonium-hydrogensulfat werden 18 mL 50%ige wässrige Natronlauge zugegeben, 2 Stunden im Eis und über Nacht bei Raumtemperatur gerührt. Nach Zugabe von 200 mL Wasser und 200 mL Dichlormethan wird die organische Phase noch einmal mit Wasser gewaschen, über Natriumsulfat getrocknet, filtriert und das Filtrat eingeengt. Der Rückstand wird mit einem Dichlormethan /Essigester-Gradienten an Kieselgel chromatographiert und die Fraktionen mit dem gewünschten Alkylierungsprodukt vereinigt und eingeengt. Das verbleibende Öl wird 10 mL Essigester gelöst. Nach Zugabe von 14 mg (0,06 mmol) Ruthenium(III)-chloridhydrat wird eine Lösung von 0,32 g (1,5 mmol) Natriumperiodat in 4 ml Wasser zugegeben, über Nacht gerührt, mit Essigester verdünnt, die organische Phase mit Wasser gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 394 mg (21 %)
Elementaranalyse:

| | | | | |
|---|---|---|---|---|
| ber.: | C 43.51 | H 6.01 | I 27.04 | N 2.98 |
| gef: | C 43.22 | H 6.34 | I 26.59 | N 3.04 |

b) 4-(6-Fluorohexyloxy)-5-oxo-pyrrolidin-1,2-disäure 1-tert-butyl ester 2-methyl ester 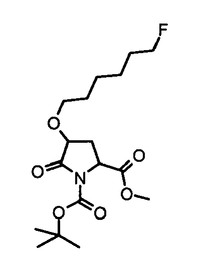 Zu 0.47 g (1 mmol) der in Beispiel **4a** beschriebenen Verbindung in 30 mL Acetonitril wird eine Lösung von 152 mg (1,12 mmol) Silberfluorid in 1,5 mL Wasser zugegeben und über Nacht bei 40°C gerührt. Die erhaltene Suspension wird filtriert, die Lösung zur Trockne eingedampft und das so erhaltene Rohprodukt wird mit einem Hexan/Essigester-Gradienten an Kieselgel chromatographiert und die entsprechenden Fraktionen vereinigt und eingeengt.
Ausbeute: 90 mg (25 %)
Elementaranalyse:

| | | | | |
|---|---|---|---|---|
| ber.: | C 56.50 | H 7.81 | F 5.26 | N 3.88 |
| gef: | C 56.25 | H 8.02 | F 5.11 | N 3.67 |

c) 2-Amino-4-(6-fluorohexyloxy)-pentandisäure 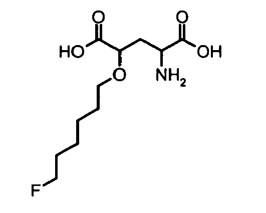 25,3 mg (0,07 mmol) der in Beispiel **4c** beschriebenen Verbindung werden in 2 mL THF gelöst, mit 1 mL 1 N Natronlauge versetzt und 4 h bei Raumtemperatur gerührt. Anschließend wird zur Trockne eingeengt und das erhaltene Rohprodukt in ca. 20 mL 6 N wässriger Salzsäure suspendiert, 6 h bei 80°C gerührt und eingeengt. Das so erhaltene Rohprodukt wird mit einem Wasser/Methanol-Gradienten an C18-Kieselgel chromatographiert und die entsprechenden Fraktionen vereinigt und eingeengt.
Ausbeute: 3,5 mg (19 %)
Elementaranalyse (berechnet auf die wasserfreie Verbindung):

| | | | | |
|---|---|---|---|---|
| ber.: | C 49.80 | H 7.60 | F 7.16 | N 5.28 |
| gef: | C 49.57 | H 7.80 | F 6.96 | N 5.33 |

d) 4-(6-[F-18]fluoro-hexyloxy)-5-oxo-pyrrolidin-1,2-disäure 1-tert-butyl ester 2-methyl ester 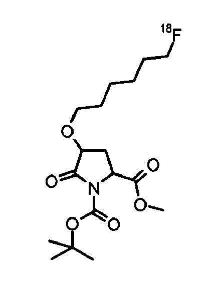 [F-18]Fluorid wurde über die [O-18](p,n)[F-18]-Reaktion in einem Zyklotron hergestellt. Die Isotopenlösung (3,8 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120°C in einem Stickstoffstrom unter Zugabe von Acetonitril (dreimal 1 mL) entfernt.
5 mg (10.7 µmol) 4-(6-lodohexyloxy)-5-oxo-pyrrolidin-1,2-disäure 1-tert-butyl ester 2-methyl ester **4a** in 1 mL Acetonitril wurden zugegeben und die resultierende Mischung wurde 10 min bei 100 °C gerührt. Nach Abkühlen auf ca. 60°C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 80 min wurden 1,0 GBq (44 % d.c.) 4-(6-[F-18]Fluorohexyloxy)-5-oxo-pyrrolidin-1,2-disäure 1-tert-butyl ester 2-methyl ester **4d** erhalten.
e) 2-Amino-4-(6-[F-18]fluorohexyloxy)-pentandisäure 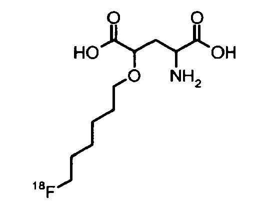 1,0 GBq 4-(6-[F-18]Fluorohexyloxy)-5-oxo-pyrrolidin-1,2-disäure 1-tert-butyl ester 2-methyl ester **4d** in 1 mL Acetonitril wurden mit 0.5 mL 4N HCl versetzt. Die Mischung wurde für 10 min unter Rühren auf 130°C (Ölbadtemperatur) erhitzt. Nach Abkühlen auf Raumtemperatur wurde die Lösung durch Zugabe von ca. 700 µL 2N NaOH neutralisiert.
Es wurden 900 MBq (90 % d.c.) 2-Amino-4-(6-[F-18]fluorohexyloxy)-pentandisäure **4e** erhalten.

### Beispiel 5

### Biologische Charakterisierung:

Die Aufnahme der erfindungsgemäßen Glutaminsäurederivate in Tumorzellen wurde in Zellexperimenten untersucht. Dabei wurde die Aufnahme eines radiomarkierten Glutaminsäurederivates (4R/S-[F-18]F-L-Glutaminsäure) in Gegenwart der erfindungsgemäßen Verbindungen untersucht (Kompetitionsexperimente). Die erfindungsgemäßen Verbindungen wurden im Überschuss (1 mM) eingesetzt zu 4R/S-[F-18]F-L-Glutaminsäure (Tracer).
Als Kontrolle wurde native L-konfigurierte Glutaminsäure (L-Glu) verwendet, welche in Konzentration von 1 mM L-Glu im Assay eine 87 % ige Inhibierung der Traceraufnahme bedingt.

Überraschenderweise wurde gefunden, dass auch 4-(6-Fluorohexyl)-L-Glu zu einer 94 % igen Inhibierung der 4R/S-[F-18]F-L-Glutaminsäure-(Tracer)-Aufnahme führt. Kompetition mit 4-(8-Fluorooctyl)-L-Glu führt ebenfalls zu einer 94 %igen Inhibierung der Traceraufnahme.

**Tabelle1: Aufnahmeinhibition der 4R/S-[F-18]F-L-Glutaminsäure durch Kompetition mit erfindungsgemäßen Verbindungen im Kompetitionszellexperiment. (A549 Zellen, 10 min Inkubation mit 0.250 MBq 4R/S-[F-18]F-L-Glu in PBS-Puffer, Kompetitorkonzentration jeweils 1 mM)**

| | **% Tracer-** | |
|---|---|---|
| | **Aufnahme** | S.D. |
| **Kontrolle** | 100.0 | 3.5 |
| **L-Glu** | 12.6 | 1.6 |
| **4-(R/S)-Fluoro-D/L-Glu** | 16.3 | 2.5 |
| **(4S)-Hydroxy-Glu** | 13.2 | 1.8 |
| **(4S)-Methyl-Glu** | 7.7 | 1.9 |
| **(4S)-4-(3-Fluoropropyl)-Glu** | 5.4 | 2.2 |
| **4-(6-Fluorohexyl)-Glu** | 6.3 | 2.4 |
| **4-(Fluorocyclobutyl)-Glu** | 5.7 | 1.3 |

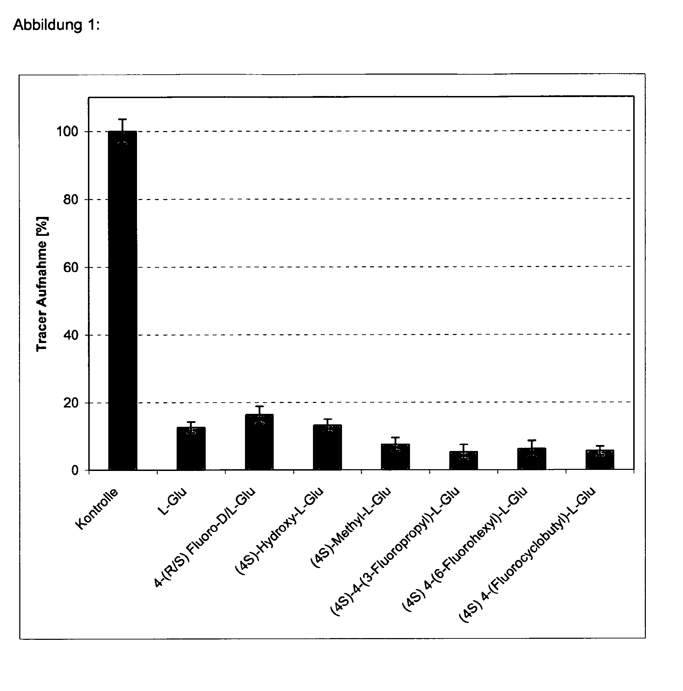

Abbildung 1: Graphische Darstellung der Inhibition von 4R/S-[F-18]F-L-Glu-Zellaufnahme durch in 4-Position verzweigte erfindungsgemäße Verbindungen in A549-Zellen (humanes nichtkleinzelliges Bronchialkarzinom) nach 10 min. Inkubation.

Nach F-18-Markierung wurde 4-(6-[F-18]Fluorohexyl)-L-Glu in Zellexperimenten an A549-Tumorzellen (humanes nicht-kleinzelliges Bronchialkarzinom) untersucht. Dabei konnte eine zeitliche Abhängigkeit der Zellaufnahme beobachtet werden.
Nach 30 min Inkubation wurde eine Aufnahme von 809000 cpm pro 100.000 Zellen für 4-(6-[F-18]Fluorohexyl)-L-Glu gemessen. Die Anreicherung liegt damit höher als für [F-18]FDG.

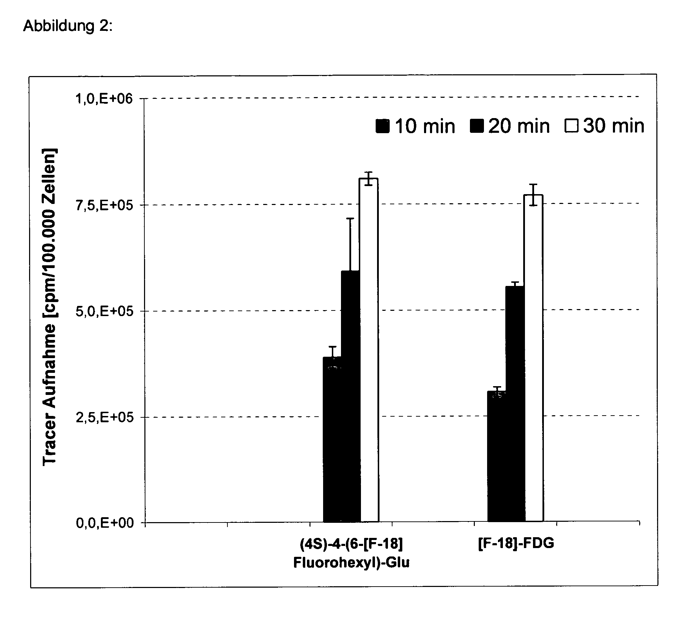

Abbildung 2:. Zeitabhängige Zellaufnahme von 4-(6-[F-18]Fluorohexyl)-L-Glu im Vergleich mit [F-18]FDG. Für alle F-18 markierten Verbindungen wurde eine zeitliche Abhängigkeit der intrazellulären Radioaktivität beobachtet. Nach 30 min wurden 809000 cpm /100000 Zellen von 4-(6-[F-18]Fluorohexyl)-L-Glu aufgenommen. Von [F-18]FDG wurden 780000 cpm/100000 Zellen nach 30 min aufgenommen.

## Patentansprüche

1. Verbindungen der allgemeinen Formel I 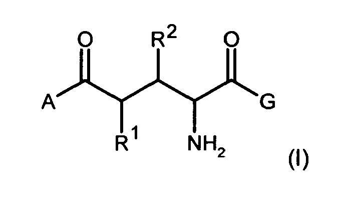 worin
A für
a) Hydroxyl,
b) verzweigtes oder unverzweigtes C₁-C₅ Alkoxy,
c) verzweigtes oder unverzweigtes Hydroxy C₁-C₅ Alkoxy,
d) verzweigtes oder unverzweigtes O-C₁-C₅ Alkyl-(O-C₁-C₄Alkyl)ₙ-O-C₁-C₄ Alkyl,
e) N(C₁-C₅ Alkyl)₂,
f) NH₂,
g) N(H)-L,
h) O-L oder
i) O-Z
steht,
G für
a) Hydroxyl,
b) O-Z
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄Alkyl)ₙO-C₁-C₄ Alkyl,
f) verzweigtes oder unverzweigtes O-C₂-C₅ Alkinyl, oder
g) Triphenylmethoxy
steht,
R¹ und R² für
i) Wasserstoff,
j) verzweigtes oder unverzweigtes ¹⁸F-C₆-C₁₀ Alkoxy,
k) verzweigtes oder unverzweigtes ¹⁸F-C₆-C₁₀ Alkyl,
l) verzweigtes oder unverzweigtes ¹⁸F-C₆-C₁₀ Alkenyl,
m) verzweigtes oder unverzweigtes ¹⁸F-C₆-C₁₀ Alkinyl,
n) Hydroxyl,
o) verzweigtes oder unverzweigtes C₁-C₅ Alkyl oder
p) verzweigtes oder unverzweigtes C₁-C₅ Alkoxy
steht,
mit der Maßgabe, dass einer der Substituenten R¹ oder R² genau ein ¹⁸F Isotop beinhaltet und der jeweils andere Substituent kein ¹⁸F Isotop beinhaltet,
L für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ Alkyl)ₙ-O-C₁-C₄ Alkyl oder
d) verzweigtes oder unverzweigtes C₂-C₅ Alkinyl,
steht, und
Z für ein Metallkationenequivalent steht,
wobei
n = 0, 1, 2 oder 3 ist und
alle Diastereomere und Enantiomere umfasst sind sind.

2. Verbindungen nach Anspruch 1, **dadurch gekennzeichnet, dass** A für Hydroxyl, verzweigtes oder unverzweigtes C₁-C₅ Alkoxy oder NH₂ steht.

3. Verbindungen nach Ansprüchen 1 oder 2, **dadurch gekennzeichnet, dass** A für NH₂ steht.

4. Verbindungen nach Ansprüchen 1 bis 3, **dadurch gekennzeichnet, dass** R¹ und R² ausgewählt ist aus der Gruppe Wasserstoff, ¹⁸F-Hexoxy, ¹⁸F-Heptoxy, ¹⁸F-Octoxy, ¹⁸F-Nonoxy, ¹⁸F-Decoxy, ¹⁸F-Hexyl, ¹⁸F-Heptyl, ¹⁸F-Octyl, ¹⁸F-Nonyl, und ¹⁸F-Decyl mit der Maßgabe, dass einer der Substituenten R¹ oder R² genau ein ¹⁸F Isotop beinhaltet und der jeweils andere Substituent Wasserstoff ist.

5. Verbindungen nach Ansprüchen 1 bis 4, **dadurch gekennzeichnet, dass** G ausgewählt ist aus der Gruppe Hydroxyl, verzweigtes oder unverzweigtes O-C₁-C₄ Alkyl.

6. Verbindungen nach Ansprüchen 1 bis 5, **dadurch gekennzeichnet, dass** G Methoxy ist.

7. Verbindungen nach Ansprüchen 1 bis 6, **dadurch gekennzeichnet, dass** Z ausgewählt ist aus der Gruppe Mg²⁺, Ca²⁺, Na⁺ und K⁺

8. Verbindungen nach Anspruch 1 ausgewählt aus der Gruppe von Verbindungen mit der Formel:

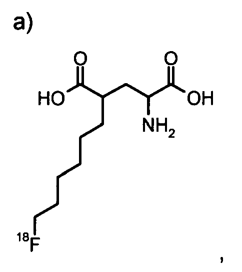

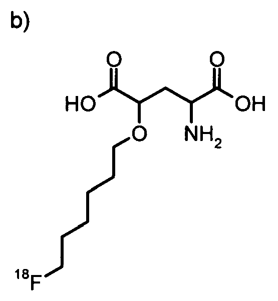

9. Verbindungen gemäß den Ansprüchen 1 bis 8 zur Verwendung als Arzneimittel.

10. Verbindungen gemäß den Ansprüchen 1 bis 8 zur Verwendung für die Bildgebung bei Tumorerkrankungen.

11. Verwendung von Verbindungen gemäß den Ansprüchen 1 bis 8 zur Herstellung eines Arzneimittels für die Bildgebung bei Tumorerkrankungen.

12. Verfahren zur Herstellung von Verbindungen der allgemeinen Formel (I) gemäß Ansprüchen 1 bis 8
indem
- eine oder mehrere vorhandene Schutzgruppen einer Verbindung nach Formel (II) gemäß Ansprüchen 13 bis 22 abgespalten werden.

13. Verbindungen der allgemeinen Formel (II): 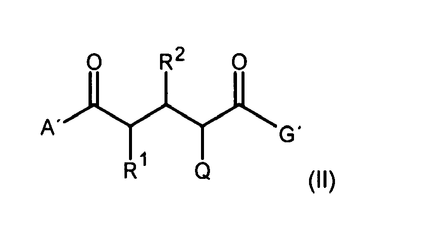 worin,
A' für
a) Hydroxyl,
b) verzweigtes oder unverzweigtes C₁-C₅ Alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ Alkyl)ₙ-O-C₁-C₄ Alkyl,
e) N(C₁-C₅ Alkyl)₂,
f) NH₂,
g) N(H)-L', oder
h) O-L'
steht,
G' für
a) hydroxyl,
b) O-Z',
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ Alkyl)ₙ-O-C₁-C₄ Alkyl,
f) verzweigtes oder unverzweigtes O-C₂-C₅ Alkinyl oder
g) Triphenylmethoxy
steht,
R¹ und R² für
a) Wasserstoff,
b) verzweigtes oder unverzweigtes ¹⁸F-C₆-C₁₀ Alkoxy,
c) verzweigtes oder unverzweigtes ¹⁸F-C₆-C₁₀ Alkyl,
d) verzweigtes oder unverzweigtes ¹⁸F-C₆-C₁₀ Alkenyl,
e) verzweigtes oder unverzweigtes ¹⁸F-C₆-C₁₀ Alkinyl,
f) Hydroxyl,
g) verzweigtes oder unverzweigtes C₁-C₅ Alkyl oder
h) verzweigtes oder unverzweigtes C₁-C₅ Alkoxy
steht,
mit der Maßgabe, dass genau einer der Substituenten R¹ oder R² genau ein ¹⁸F Isotop und der jeweils andere Substituent kein ¹⁸F Isotop beinhaltet,
Q für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
e) N(H)-2,2,2-Trichlorethoxycarbonyl,
f) N(H)-1,1-Dimethylpropinyl,
g) N(H)-1-Methyl-1-phenyl-ethoxycarbonyl,
h) N(H)-1-Methyl-1-(4-biphenylyl)-ethoxycarbonyl,
i) N(H)-cyclobutylcarbonyl,
j) N(H)-1-Methylcyclobutylcarbonyl,
k) N(H)-Vinylcarbonyl,
l) N(H)-Allylcarbonyl,
m) N(H)-Adamantylcarbonyl,
n) N(H)-Diphenylmethylcarbonyl,
o) N(H)-Cinnamylcarbonyl,
p) N(H)-Formyl,
q) N(H)-Benzoyl,
r) N(H)-Trityl,
s) N(H)-p-Methoxyphenyl-diphenylmethyl,
t) N(H)-di-(p-Methoxyphenyl)-phenylmethyl,
u) 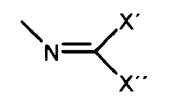 oder
v) N-(tert-Butoxycarbonyl)₂,
steht,
L' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
X' und X'' unabhängig voneinander für
a) verzweigtes oder unverzweigtes C₁-C₅ Alkyl,
b) substituiertes oder unsubstituiertes Aryl,
c) substituiertes oder unsubstituiertes Aralkyl, oder
d) substituiertes oder unsubstituiertes heteroaryl
steht,
Z' für ein Metallkationenequivalent
steht,
wobei n = 0, 1, 2 oder 3 ist und
alle Diastereomere und Enantiomere umfasst sind sind.

14. Verbindungen nach Anspruch 13, **dadurch gekennzeichnet, dass** A' für Hydroxyl, verzweigtes oder unverzweigtes C₁-C₅ Alkoxy, oder NH₂ steht.

15. Verbindungen nach Ansprüch 14, **dadurch gekennzeichnet, dass** A' für NH₂ steht.

16. Verbindungen nach Ansprüchen 13 bis 15, **dadurch gekennzeichnet, dass** R¹ und R² ausgewählt ist aus der Gruppe Wasserstoff, ¹⁸F-Hexoxy, ¹⁸F-Heptoxy, ¹⁸F-Octoxy, ¹⁸F-Nonoxy, ¹⁸F-Decoxy, ¹⁸F-Hexyl, ¹⁸F-Heptyl, ¹⁸F-Octyl, ¹⁸F-Nonyl, und ¹⁸F-Decyl mit der Maßgabe, dass einer der Substituenten R¹ oder R² genau ein ¹⁸F Isotop beinhaltet und der jeweils andere Substituent Wasserstoff ist.

17. Verbindungen nach Ansprüchen 13 bis 16, **dadurch gekennzeichnet, dass** G' ausgewählt ist aus der Gruppe Hydroxyl, verzweigtes oder unverzweigtes C₁-C₄ Alkoxy und OZ'.

18. Verbindungen nach Ansprüchen 13 bis 17, **dadurch gekennzeichnet, dass** G' Methoxy ist.

19. Verbindungen nach Ansprüchen 13 bis 18, **dadurch gekennzeichnet, dass** Z' ausgewählt ist aus der Gruppe Na⁺, K⁺, Ca²⁺ und Mg²⁺.

20. Verbindungen nach Ansprüchen 13 bis19, **dadurch gekennzeichnet, dass** Q ausgewählt ist aus der Gruppe N(H)-tert-Butoxycarbonyl, N(H)-Benzyloxycarbonyl und 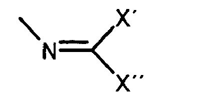 worin
X' und X'' unabhängig voneinander für
a) verzweigtes oder unverzweigtes C₁-C₅ Alkyl,
b) substituiertes oder unsubstituiertes Aryl,
c) substituiertes oder unsubstituiertes Aralkyl, oder
d) substituiertes oder unsubstituiertes heteroaryl
steht.

21. Verbindungen nach Ansprüchen 13 bis 20 **dadurch gekennzeichnet, dass** Q für N(H)-tert-Butoxycarbonyl oder N(H)-Trityl steht.

22. Verbindungen nach Anspruch 13 ausgewählt aus der Gruppe von Verbindungen mit der Formel:

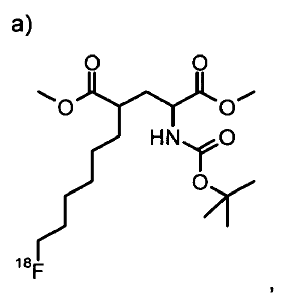

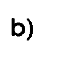

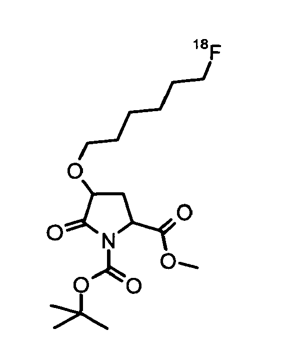

23. Verbindungen gemäß den Ansprüchen 13 bis 22 zur Verwendung als Arzneimittel.

24. Verbindungen gemäß den Ansprüchen 13 bis 22 zur Verwendung für die Bildgebung bei Tumorerkrankungen.

25. Verwendung von Verbindungen gemäß den Ansprüchen 1 bis 22 zur Herstellung eines Arzneimittels für die Bildgebung bei Tumorerkrankungen.

26. Verfahren zur Herstellung von Verbindungen der allgemeinen Formel (II) gemäß Ansprüchen 13 bis 22
indem
- eine Verbindung nach Formel (III) gemäß Anspruch 27 mit F-18 Fluorid umgesetzt wird.

27. Verbindungen der Formel (III) 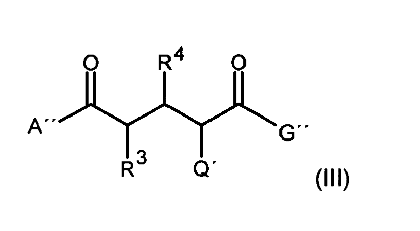 worin
A'' für
a) verzweigtes oder unverzweigtes C₁-C₅ Alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl,
d) N(C₁-C₅ Alkyl)₂,
e) NH₂,
f) N(H)-L" oder
g) O-L''
steht,
G'' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) Triphenylmethoxy
steht,
R³ und R⁴ für
i) Wasserstoff,
j) verzweigtes oder unverzweigtes E-C₆-C₁₀ Alkoxy,
k) verzweigtes oder unverzweigtes E-C₆-C₁₀ Alkyl,
l) verzweigtes oder unverzweigtes E-C₆-C₁₀ Alkenyl,
m) verzweigtes oder unverzweigtes E-C₆-C₁₀ Alkinyl,
n) Hydroxyl,
o) verzweigtes oder unverzweigtes C₁-C₅ Alkyl oder
p) verzweigtes oder unverzweigtes C₁-C₅ Alkoxy
steht, mit der Maßgabe, dass genau einer der Substituenten R³ oder R⁴ ein E beinhaltet und der jeweils andere Substituent kein E beinhaltet,
E für
a) Chloro,
b) Bromo,
c) Mesyloxy,
d) Trifluoromethylsulfonyloxy,
e) Nonafluorbutyloxy,
f) Tosyloxy, oder
g) lodo
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 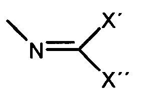 oder
x) N-(tert-Butoxycarbonyl)₂,
steht,
L'' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
X' and X'' unabhängig voneinander für
a) verzweigtes oder unverzweigtes C₁-C₅ Alkyl,
b) substituiertes oder unsubstituiertes Aryl
c) substituiertes oder unsubstituiertes Alkylaryl oder
d) substituiertes oder unsubstituiertes Heteroaryl
steht, und
wobei n = 0, 1, 2 oder 3 ist und
alle Diastereomere und Enantiomere umfasst sind.

28. Verwendung von Verbindungen der Formel (III) zur Herstellung von Verbindungen der Formel (I) oder (II): 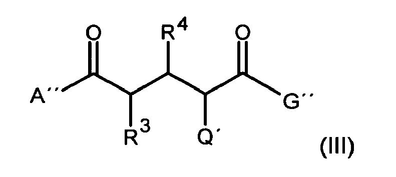 worin
A'' für
a) verzweigtes oder unverzweigtes C₁-C₅ Alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl,
d) N(C₁-C₅ Alkyl)₂,
e) NH₂,
f) N(H)-L" oder
g) O-L''
steht,
G'' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) Triphenylmethoxy
steht,
R³ und R⁴ für
a) Wasserstoff,
b) verzweigtes oder unverzweigtes E-C₆-C₁₀ Alkoxy,
c) verzweigtes oder unverzweigtes E-C₆-C₁₀ Alkyl,
d) verzweigtes oder unverzweigtes E-C₆-C₁₀ Alkenyl,
e) verzweigtes oder unverzweigtes E-C₆-C₁₀ Alkinyl,
f) Hydroxyl,
g) verzweigtes oder unverzweigtes C₁-C₅ Alkyl oder
h) verzweigtes oder unverzweigtes C₁-C₅ Alkoxy
steht, mit der Maßgabe, dass genau einer der Substituenten R³ oder R⁴ ein E beinhaltet und der jeweils andere Substituent kein E beinhaltet,
E für
a) Chloro,
b) Bromo,
c) Methansulfonyloxy,
d) Trifluormethansulfonyloxy,
e) Nonafluorbutyloxy,
f) Tosyloxy, oder
g) lodo
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 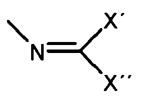 oder
x) N-(tert-Butoxycarbonyl)₂,
steht,
L'' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
X' and X'' unabhängig voneinander für
a) verzweigtes oder unverzweigtes C₁-C₅ Alkyl,
b) substituiertes oder unsubstituiertes Aryl
c) substituiertes oder unsubstituiertes Alkylaryl oder
d) substituiertes oder unsubstituiertes Heteroaryl
steht, und
wobei n = 0, 1, 2 oder 3 ist und
alle Diastereomere und Enantiomere umfasst sind.

29. Verbindungen der Formel (IV) 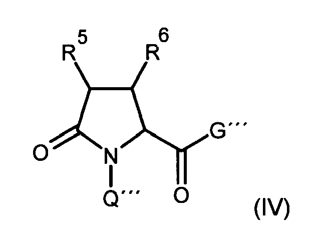 worin
G"' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) triphenylmethoxy
steht,
R⁵ und R⁶ für
a) Wasserstoff,
b) Hydroxyl,
c) verzweigtes oder unverzweigtes C₁-C₅ Alkyl,
d) verzweigtes oder unverzweigtes C₁-C₅ Alkoxy oder
e) R⁷-E'
steht, mit der Maßgabe, dass genau einer der Substituenten R⁵ oder R⁶ ein E' beinhaltet und der jeweils andere Substituent kein E' beinhaltet,
E' für
a) Chloro,
b) Bromo,
c) Methansulfonyloxy,
d) Trifluormethansulfonyloxy,
e) Nonafluorbutyloxy,
f) Tosyloxy oder
g) lodo
steht,
R⁷ für
a) verzweigtes oder unverzweigtes C₆-C₁₀ Alkoxy,
b) verzweigtes oder unverzweigtes C₆-C₁₀ Alkyl,
c) verzweigtes oder unverzweigtes C₆-C₁₀ Alkenyl, oder
d) verzweigtes oder unverzweigtes C₆-C₁₀ Alkinyl
steht,
Q''' für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 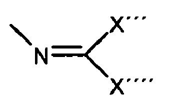 oder
x) N-(tert-Butoxycarbonyl)₂,
steht,
X''' and X'''' unabhängig voneinander für
a) verzweigtes oder unverzweigtes C₁-C₅ Alkyl,
b) substituiertes oder unsubstituiertes Aryl,
c) substituiertes oder unsubstituiertes Alkylaryl, oder
d) substituiertes oder unsubstituiertes Heteroaryl
steht,
wobei n = 0, 1, 2 oder 3 ist und
alle Diastereomere und Enantiomere umfasst sind.

30. Verwendung von Verbindungen der Formel (IV) zur Herstellung von Verbindungen der Formel (I) oder (II): 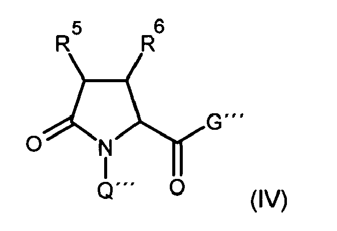 worin
G''' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) Triphenylmethoxy
steht,
R⁵ und R⁶ für
a) Wasserstoff,
b) Hydroxyl,
c) verzweigtes oder unverzweigtes C₁-C₅ Alkyl,
d) verzweigtes oder unverzweigtes C₁-C₅ Alkoxy oder
e) R⁷-E'
steht, mit der Maßgabe, dass genau einer der Substituenten R⁵ oder R⁶ ein E' beinhaltet und der jeweils andere Substituent kein E' beinhaltet,
E'für
a) Chloro,
b) Bromo,
c) Methansulfonyloxy,
d) Trifluormethansulfonyloxy,
e) Nonafluorbutyloxy,
f) Tosyloxy oder
g) lodo
steht,
R⁷ für
a) verzweigtes oder unverzweigtes C₆-C₁₀ Alkoxy,
b) verzweigtes oder unverzweigtes C₆-C₁₀ Alkyl,
c) verzweigtes oder unverzweigtes C₆-C₁₀ Alkenyl, oder
d) verzweigtes oder unverzweigtes C₆-C₁₀ Alkinyl
steht,
Q''' für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 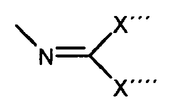 oder
x) N-(tert-Butoxycarbonyl)₂,
steht,
X''' and X'''' unabhängig voneinander für
a) verzweigtes oder unverzweigtes C₁-C₅ Alkyl,
b) substituiertes oder unsubstituiertes Aryl,
c) substituiertes oder unsubstituiertes Alkylaryl, oder
d) substituiertes oder unsubstituiertes Heteroaryl
steht,
wobei n = 0, 1, 2 oder 3 ist und
alle Diastereomere und Enantiomere umfasst sind.

31. Bildgebungs-kit enthaltend Verbindungen der allgemeinen Formel III oder IV.

32. Pharmazeutische Zusammensetzung enthaltend Verbindungen der allgemeinen Formel I, II, III oder IV und geeignete pharmazeutische Träger Substanzen.

33. Verbindungen der allgemeinen Formel V 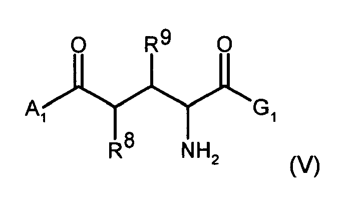 worin
A₁ für
a) Hydroxyl
b) verzweigtes oder unverzweigtes C₁-C₅ Alkoxy,
c) verzweigtes oder unverzweigtes Hydroxy C₁-C₅ Alkoxy,
d) verzweigtes oder unverzweigtes O-C₁-C₅ Alkyl-(O-C₁-C₄Alkyl)ₙ-O-C₁-C₄Alkyl,
ed) N(C₁-C₅Alkyl)₂,
f) NH₂,
g) N(H)-L₁,
h) O-L₁ oder
i) O-Z₁
steht,
G₁ für
a) Hydroxyl
b) O-Z₁
c) verzweigtes oder unverzweigtes O-C₁-C₅ Alkyl,
c) verzweigtes oder unverzweigtes O-C₂-C₅ Alkenyl,
e) verzweigtes oder unverzweigtes O-C₁-C₅ Alkyl-(O-C₁-C₄ Alkyl)ₙ-O-C₁-C₄ Alkyl,
f) verzweigtes oder unverzweigtes O-C₂-C₅ Alkinyl, oder
g) Triphenylmethoxy
steht,
R⁸ und R⁹ für
a) Wasserstoff,
b) substituiertes oder unsubstituiertes ¹⁸F-C₆-C₁₀ mono- oder bicyclisches Aryl,
c) substituiertes oder unsubstituiertes ¹⁸F-C₅-C₁₀ mono- oder bicyclisches Heteroaryl,
d) substituiertes oder unsubstituiertes ¹⁸F-C₃-C₆ Cyclo-Alkyl,
e) Hydroxyl,
f) verzweigtes oder unverzweigtes C₁-C₅ Alkyl oder
g) verzweigtes oder unverzweigtes C₁-C₅ Alkoxy
steht,
mit der Maßgabe, dass einer der Substituenten R⁸ oder R⁹ genau ein ¹⁸F Isotop beinhaltet und der jeweils andere Substituent kein ¹⁸F Isotop beinhaltet,
L₁ für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄Alkyl)ₙ-O-C₁-C₄Alkyl oder
d) verzweigtes oder unverzweigtes C₂-C₅ Alkinyl,
steht,
Z₁ für ein Metallkationenequivalent steht,
wobei
n = 0, 1, 2 oder 3 ist und
alle Diastereomere und Enantiomere umfasst sind.

34. Verbindungen nach Anspruch 33, **dadurch gekennzeichnet, dass** A₁ für Hydroxyl, verzweigtes oder unverzweigtes C₁-C₅ Alkoxy oder NH₂ steht.

35. Verbindungen nach Ansprüchen 33 oder 34, **dadurch gekennzeichnet, dass** A₁ für NH₂ steht.

36. Verbindungen nach Ansprüchen 33 bis 35, **dadurch gekennzeichnet, dass** R⁸ und R⁹ ausgewählt ist aus der Gruppe Wasserstoff, substituiertes oder unsubstituiertes ¹⁸F-C₆-C₁₀ Aryl, substituiertes oder unsubstituiertes ¹⁸F-C₅-C₁₀ Heteroaryl, und substituiertes oder unsubstituiertes ¹⁸F-C₃-C₆ Cyclo-Alkyl, mit der Maßgabe, dass einer der Substituenten R⁸ oder R⁹genau ein ¹⁸F Isotop beinhaltet und der jeweils andere Substituent Wasserstoff ist.

37. Verbindungen nach Ansprüchen 33 bis 36, **dadurch gekennzeichnet, dass** G₁ ausgewählt ist aus der Gruppe Hydroxyl, verzweigtes oder unverzweigtes O-C₁-C₄ Alkyl.

38. Verbindungen nach Ansprüch 37, **dadurch gekennzeichnet, dass** G₁ Methoxy ist.

39. Verbindungen nach Ansprüchen 33 bis 38, **dadurch gekennzeichnet, dass** Z₁ ausgewählt ist aus der Gruppe Mg²⁺, Ca²⁺, Na⁺ und K⁺

40. Verbindungen nach Anspruch 33 ausgewählt aus der Gruppe von Verbindungen mit der Formel:

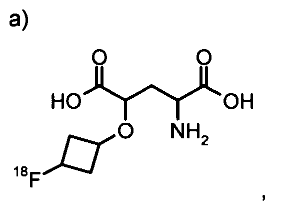

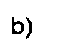

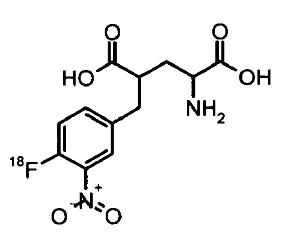

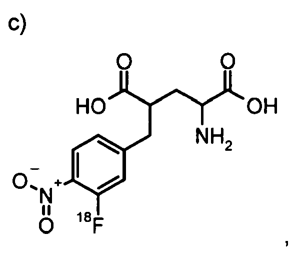

41. Verbindungen gemäß den Ansprüchen 33 bis 40 zur Verwendung als Arzneimittel.

42. Verbindungen gemäß den Ansprüchen 33 bis 40 zur Verwendung für die Bildgebung bei Tumorerkrankungen.

43. Verwendung von Verbindungen gemäß den Ansprüchen 33 bis 40 zur Herstellung eines Arzneimittels für die Bildgebung bei Tumorerkrankungen.

44. Verfahren zur Herstellung von Verbindungen der allgemeinen Formel (V) gemäß Ansprüchen 33 bis 40
indem
- eine oder mehrere vorhandene Schutzgruppen einer Verbindung nach Formel (VI) gemäß Ansprüchen 45 bis 54 abgespalten werden..

45. Verbindungen der allgemeinen Formel (VI): 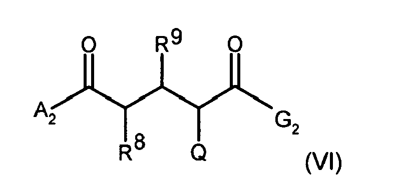 worin,
A₂ für
a) Hydroxyl,
b) verzweigtes oder unverzweigtes C₁-C₅ Alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ Alkyl)ₙ-O-C₁-C₄ Alkyl,
e) N(C₁-C₅ Alkyl)₂,
f) NH₂,
g) N(H)-L₂, oder
h) O-L₂
steht,
G₂ für
a) hydroxyl,
b) O-Z₂,
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ Alkyl)ₙ-O-C₁-C₄ Alkyl,
f) verzweigtes oder unverzweigtes O-C₂-C₅ Alkinyl oder
g) triphenylmethoxy steht,
R₈ und R₉ für
a) Wasserstoff,
b) substituiertes oder unsubstituiertes ¹⁸F-C₆-C₁₀ mono- oder bicyclisches Aryl,
c) substituiertes oder unsubstituiertes ¹⁸F-C₅-C₁₀ mono- oder bicyclisches Heteroaryl,
d) substituiertes oder unsubstituiertes ¹⁸F-C₃-C₆ Cyclo-Alkyl,
e) Hydroxyl,
f) verzweigtes oder unverzweigtes C₁-C₅ Alkyl oder
g) verzweigtes oder unverzweigtes C₁-C₅ Alkoxy steht,
mit der Maßgabe, dass genau einer der Substituenten R₈ oder R⁹ genau ein ¹⁸F Isotop und der jeweils andere Substituent kein ¹⁸F Isotop beinhaltet,
Q₁ für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
e) N(H)-2,2,2-Trichlorethoxycarbonyl,
f) N(H)-1,1-Dimethylpropinyl,
g) N(H)-1-Methyl-1-phenyl-ethoxycarbonyl,
h) N(H)-1-Methyl-1-(4-biphenylyl)-ethoxycarbonyl,
i) N(H)-cyclobutylcarbonyl,
j) N(H)-1-Methylcyclobutylcarbonyl,
k) N(H)-Vinylcarbonyl,
l) N(H)-Allylcarbonyl,
m) N(H)-Adamantylcarbonyl,
n) N(H)-Diphenylmethylcarbonyl,
o) N(H)-Cinnamylcarbonyl,
p) N(H)-Formyl,
q) N(H)-Benzoyl,
r) N(H)-Trityl,
s) N(H)-p-Methoxyphenyl-diphenylmethyl,
t) N(H)-di-(p-Methoxyphenyl)-phenylmethyl,
u) 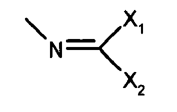 oder
v) N-(tert-Butoxycarbonyl)₂,
steht,
L₂ für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
X₁ und X₂ unabhängig voneinander für
a) verzweigtes oder unverzweigtes C₁-C₅ Alkyl,
b) substituiertes oder unsubstituiertes Aryl,
c) substituiertes oder unsubstituiertes Aralkyl, oder
d) substituiertes oder unsubstituiertes heteroaryl
steht, und
Z₂ für ein Metallkationenequivalent
steht,
wobei n = 0, 1, 2 oder 3 ist und
alle Diastereomere und Enantiomere umfasst sind.

46. Verbindungen nach Anspruch 45, **dadurch gekennzeichnet, dass** A2 für Hydroxyl, verzweigtes oder unverzweigtes C₁-C₅ Alkoxy, oder NH₂ steht.

47. Verbindungen nach Anspruch 46, **dadurch gekennzeichnet, dass** A2 für NH₂ steht.

48. Verbindungen nach Ansprüchen 45 bis 47, **dadurch gekennzeichnet, dass** R⁸ und R⁹ ausgewählt ist aus der Gruppe Wasserstoff, substituiertes oder unsubstituiertes ¹⁸F-C₆-C₁₀ Aryl, substituiertes oder unsubstituiertes ¹⁸F-C₅-C₁₀ Heteroaryl, und substituiertes oder unsubstituiertes ¹⁸F-C₃-C₆ Cyclo-Alkyl, mit der Maßgabe, dass einer der Substituenten R⁸ oder R⁹ genau ein ¹⁸F Isotop beinhaltet und der jeweils andere Substituent Wasserstoff ist.

49. Verbindungen nach Ansprüchen 45 bis 48, **dadurch gekennzeichnet, dass** G₂ ausgewählt ist aus der Gruppe Hydroxyl, verzweigtes oder unverzweigtes C₁-C₄ Alkoxy und OZ₂.

50. Verbindungen nach Ansprüchen 45 bis 49, **dadurch gekennzeichnet, dass** G₂ Methoxy ist.

51. Verbindungen nach Ansprüchen 45 bis 50, **dadurch gekennzeichnet, dass** Z₂ ausgewählt ist aus der Gruppe Na⁺, K⁺, Ca²⁺ und Mg²⁺.

52. Verbindungen nach Ansprüchen 45 bis 51, **dadurch gekennzeichnet, dass** Q₁ ausgewählt ist aus der Gruppe N(H)-tert-Butoxycarbonyl, N(H)-Benzyloxycarbonyl und 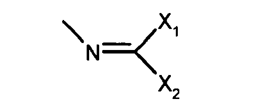 worin
X₁ und X₂ unabhängig voneinander für
a) verzweigtes oder unverzweigtes C₁-C₅ Alkyl,
b) substituiertes oder unsubstituiertes Aryl,
c) substituiertes oder unsubstituiertes Aralkyl, oder
d) substituiertes oder unsubstituiertes heteroaryl
steht.

53. Verbindungen nach Ansprüchen 45 bis 52 **dadurch gekennzeichnet, dass** Q₁ für N(H)-tert-Butoxycarbonyl oder N(H)-Trityl steht.

54. Verbindungen nach Anspruch 45:

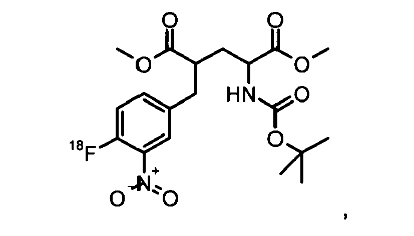

55. Verbindungen gemäß den Ansprüchen 45 bis 54 zur Verwendung als Arzneimittel.

56. Verbindungen gemäß den Ansprüchen 45 bis 54 zur Verwendung für die Bildgebung bei Tumorerkrankungen.

57. Verwendung von Verbindungen gemäß den Ansprüchen 45 bis 54 zur Herstellung eines Arzneimittels für die Bildgebung bei Tumorerkrankungen.

58. Verfahren zur Herstellung von Verbindungen der allgemeinen Formel (VI) gemäß Ansprüchen 45 bis 54
indem
- eine Verbindung nach Formel (VII) gemäß Anspruch 59 mit F-18 Fluorid umgesetzt wird.

59. Verbindungen der Formel (VII) 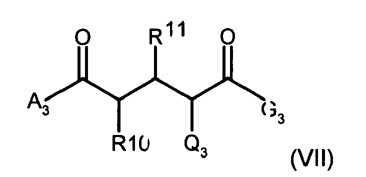 worin
A₃ für
a) verzweigtes oder unverzweigtes C₁-C₅ Alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl
d) N(C₁-C₅ Alkyl)₂,
e) NH₂,
f) N(H)-L₃ oder
g) O-L₃
steht,
G₃ für
a) O-Z₃,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
steht,
R¹⁰ und R¹¹ für
a) Wasserstoff,
d) substituiertes oder unsubstituiertes E₁-C₆-C₁₀ mono- oder bicyclisches Aryl,
e) substituiertes oder unsubstituiertes E₁-C₅-C₁₀ mono- oder bicyclisches Heteroaryl,
d) substituiertes oder unsubstituiertes E₁-C₃-C₆ Cyclo-Alkyl,
e) Hydroxyl,
f) verzweigtes oder unverzweigtes C₁-C₅ Alkyl oder
g) verzweigtes oder unverzweigtes C₁-C₅ Alkoxy
steht, mit der Maßgabe, dass genau einer der Substituenten R¹⁰ oder R¹¹ ein E₁ beinhaltet und der jeweils andere Substituent kein E₁ beinhaltet,
E₁ für
a) Chloro,
b) Bromo,
c) Methansulfonyloxy,
d) Trifluormethansulfonyloxy,
e) Nonafluorbutyloxy,
f) Tosyloxy, oder
g) lodo
steht,
Q₂ für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 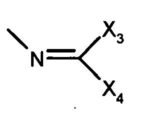 oder
x) N-(tert-Butoxycarbonyl)₂,
steht,
L₃ für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
X₃ and X₄ unabhängig voneinander für
a) verzweigtes oder unverzweigtes C₁-C₅ Alkyl,
b) substituiertes oder unsubstituiertes Aryl
c) substituiertes oder unsubstituiertes Alkylaryl oder
d) substituiertes oder unsubstituiertes Heteroaryl
steht,
Z₃ für ein Metallkationenequivalent steht,
wobei n = 0, 1, 2 oder 3 ist und
alle Diastereomere und Enantiomere umfasst sind.

60. Verwendung von Verbindungen der Formel (VII) zur Herstellung von Verbindungen der Formel (V) oder (VI): 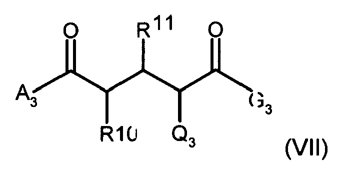 worin
A₃ für
a) verzweigtes oder unverzweigtes C₁-C₅ Alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl
d) N(C₁-C₅ Alkyl)₂,
e) NH₂,
f) N(H)-L₃ oder
g) O-L₃
steht,
G₃ für
a) O-Z₃,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
steht,
R¹⁰ und R¹¹ für
a) Wasserstoff,
b) substituiertes oder unsubstituiertes E₁-C₆-C₁₀ mono- oder bicyclisches Aryl,
c) substituiertes oder unsubstituiertes E₁-C₅-C₁₀ mono- oder bicyclisches Heteroaryl,
d) substituiertes oder unsubstituiertes E₁-C₃-C₆ Cyclo-Alkyl,
e) Hydroxyl,
f) verzweigtes oder unverzweigtes C₁-C₅ Alkyl oder
g) verzweigtes oder unverzweigtes C₁-C₅ Alkoxy
steht, mit der Maßgabe, dass genau einer der Substituenten R¹¹ oder R¹² ein E₁ beinhaltet und der jeweils andere Substituent kein E₁ beinhaltet,
E₁ für
a) Chloro,
b) Bromo,
c) Methansulfonyloxy,
d) Trifluormethansulfonyloxy,
e) Nonafluorbutyloxy,
f) Tosyloxy, oder
g) lodo
steht,
Q₂ für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 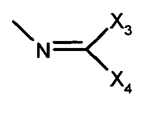 oder
x) N-(tert-Butoxycarbonyl)₂,
steht,
L₃ für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)"-O-C₁-C₄ alkyl oder
d) verzweigtes oder unverzweigtes C₂-C₅ Alkinyl
steht,
X₃ and X₄ unabhängig voneinander für
a) verzweigtes oder unverzweigtes C₁-C₅ Alkyl,
b) substituiertes oder unsubstituiertes Aryl
c) substituiertes oder unsubstituiertes Alkylaryl oder
d) substituiertes oder unsubstituiertes Heteroaryl
steht,
Z₃ für ein Metallkationenequivalent steht,
wobei n = 0, 1, 2 oder 3 ist und
alle Diastereomere und Enantiomere umfasst sind.

61. Verbindungen der Formel (VIII) 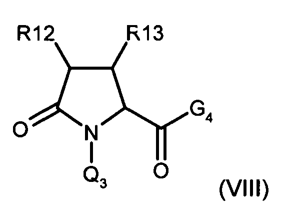 worin
G₄ für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄alkyl)ₙ-O-C₁-C₄ alkyl,
d) verzweigtes oder unverzweigtes O-C₂-C₅ Alkinyl, oder
e) triphenylmethoxy
steht,
R¹² und R¹³ für
a) Wasserstoff,
b) Hydroxyl,
c) verzweigtes oder unverzweigtes C₁-C₅ Alkyl,
d) verzweigtes oder unverzweigtes C₁-C₅ Alkoxy oder
e) R¹⁴-E₂
steht, mit der Maßgabe, dass genau einer der Substituenten R¹² oder R¹³ ein E₂ beinhaltet und der jeweils andere Substituent kein E₂ beinhaltet,
E₂ für
a) Chloro,
b) Bromo,
c) Methansulfonyloxy,
d) Trifluormethansulfonyloxy,
e) Nonafluorbutyloxy,
f) Tosyloxy oder
g) lodo
steht,
R¹⁴ für
a) substituiertes oder unsubstituiertes C₆-C₁₀ mono- oder bicyclisches Aryl,
b) substituiertes oder unsubstituiertes C₅-C₁₀ mono- oder bicyclisches Heteroaryl,
c) substituiertes oder unsubstituiertes C₃-C₆ Cyclo-Alkyl,
steht,
Q₃ für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 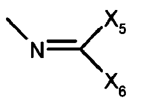 oder
x) N-(tert-Butoxycarbonyl)₂,
steht,
X₅ and X₆ unabhängig voneinander für
a) verzweigtes oder unverzweigtes C₁-C₅ Alkyl,
b) substituiertes oder unsubstituiertes Aryl
c) substituiertes oder unsubstituiertes Alkylaryl, oder
d) substituiertes oder unsubstituiertes Heteroaryl
steht,
wobei n = 0, 1, 2 oder 3 ist und
alle Diastereomere und Enantiomere umfasst sind.

62. Verwendung von Verbindungen der Formel (VIII) zur Herstellung von Verbindungen der Formel (V) oder (VI): 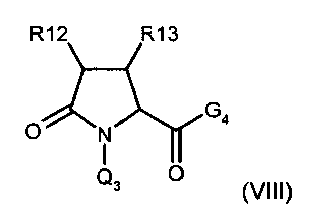 worin
G4 für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) triphenylmethoxy
steht,
R¹² und R¹³ für
a) Wasserstoff,
b) Hydroxyl,
c) verzweigtes oder unverzweigtes C₁-C₅ Alkyl,
d) verzweigtes oder unverzweigtes C₁-C₅ Alkoxy oder
e) R¹⁴-E₂
steht, mit der Maßgabe, dass genau einer der Substituenten R¹² oder R¹³ ein E₂ beinhaltet und der jeweils andere Substituent kein E₂ beinhaltet,
E₂ für
a) Chloro,
b) Bromo,
c) Methansulfonyloxy,
d) Trifluormethansulfonyloxy,
e) Nonafluorbutyloxy,
f) Tosyloxy oder
g) lodo
steht,
R₁₄ für
a) substituiertes oder unsubstituiertes C₆-C₁₀ mono- oder bicyclisches Aryl,
b) substituiertes oder unsubstituiertes C₅-C₁₀ mono- oder bicyclisches Heteroaryl,
c) substituiertes oder unsubstituiertes C₃-C₆ Cyclo-Alkyl,
steht,
Q₃ für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 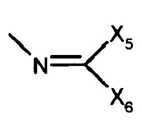 oder
x) N-(tert-Butoxycarbonyl)₂,
steht,
X₅ and X₆ unabhängig voneinander für
a) verzweigtes oder unverzweigtes C₁-C₅ Alkyl,
b) substituiertes oder unsubstituiertes Aryl
c) substituiertes oder unsubstituiertes Alkylaryl, oder
d) substituiertes oder unsubstituiertes Heteroaryl
steht,
wobei n = 0, 1, 2 oder 3 ist und
alle Diastereomere und Enantiomere umfasst sind.

63. Bildgebung-kit enthaltend Verbindungen der allgemeinen Formel VII oder VIII.

64. Pharmazeutische Zusammensetzung enthaltend Verbindungen der allgemeinen Formel V, VI, VII oder VIII und geeignete pharmazeutische Träger Substanzen.

65. Verbindungen nach Ansprüchen 1 bis 8, 13 bis 22, 33 bis 40 and 45 bis 54 **dadurch gekennzeichnet dass** die Verbindungen für Bildgebung in einem Dosisbereich von 37 - 600 MBq geeignet sind.

66. Verbindungen nach Anspruch 65 **dadurch gekennzeichnet dass** die Verbindungen in einem Dosisbereich von 150 MBq - 370 MBq besonders geeignet sind.
